Europäisches Patentamt

European Patent Office

Office européen des brevets

(19)

(11) Veröffentlichungsnummer: **0 223 247**
**A2**

(12) **EUROPÄISCHE PATENTANMELDUNG**

(21) Anmeldenummer: **86116056.2**

(22) Anmeldetag: **20.11.86**

(51) Int. Cl.4: **C12N 15/00** , **A01H 1/00** ,
**A01G 7/00** , **C12N 5/00**

(30) Priorität: **22.11.85 US 801014**

(43) Veröffentlichungstag der Anmeldung:
**27.05.87 Patentblatt 87/22**

(84) Benannte Vertragsstaaten:
**AT BE DE ES FR GR IT LU NL SE**

(71) Anmelder: **CIBA-GEIGY AG**
**Klybeckstrasse 141**
**CH-4002 Basel(CH)**

(72) Erfinder: **Potrykus, Ingo, Dr.**
**Im Stigler 54**
**CH-4312 Magden(CH)**
Erfinder: **Shillito, Raymond Douglas, Dr.**
**58 Laurel Ridge Apts.**
**Chapel Hill North Carolina 27514(US)**
Erfinder: **Chilton, Mary-Dell, Dr.**
**10513 Winding Wood Trail**
**Raleigh North Carolina 27612(US)**

(74) Vertreter: **Zumstein, Fritz, Dr. et al**
**Bräuhausstrasse 4**
**D-8000 München 2(DE)**

(54) **Direkter Gentransfer in Plastide und Mitochondrien.**

(57) Es wird ein Verfahren zur direkten Einschleusung von DNA in die Plastide und Mitochondrien von pflanzlichen Photoplasten beschrieben,
das sich im wesentlichen dadurch kennzeichnet,
dass man in Abwesenheit eines Pathogens diese
besagte DNA in einem Medium, in dem die DNA in
die Protoplasten und die in diesen befindlichen Plastide und Mitochondrien einzudringen vermag, mit
den Protoplasten so lange in Kontakt bringt, dass
diese Penetration gewährleistet ist, so dass letztlich
Pflanzen mit verbesserten Eigenschaften resultieren.

Abbildung 1

# Direkter Gentransfer in Plastide und Mitochondrien

Die vorliegende Erfindung betrifft ein Verfahren für den direkten Gentransfer in die Plastide und die Mitochondrien, vorzugsweise in die Chloroplasten von pflanzlichen Protoplasten.

Im Rahmen der vorliegenden Erfindung sind unter dem Oberbegriff Pflanzen alle ein-oder vielzelligen Organismen zu verstehen, die befähigt sind Photosynthesen durchzuführen.

Als pflanzliche Protoplasten werden Pflanzen-Zellen bezeichnet, deren Zellwände durch Behandlung mit cellulolytischen Enzymen ganz oder teilweise entfernt worden sind.

Die Chloroplasten bilden einen Angriffspunkt für Vertreter aus verschiedenen Herbizid-Klassen, so z.B. für die Triazin-Herbizide. Erst kürzlich wurde entdeckt, dass das Atrazin, ein Vertreter der Triazin-Herbizide, mit einem 32 kd Polypetid in Wechselwirkung tritt, das von einem Chloroplasten-Gen, dem sogenannten psbA-Gen, codiert wird - (Hirschberg et al., 1984).

Die Substitution eines einzigen Nucleotids innerhalb der kodierenden Region des psbA-Gens, die den Austausch einer einzigen Aminosäure im 32 kd Polypeptid zur Folge hat, führt zu einer Resistenz gegenüber Atrazin. Solche Mutationen findet man bei Unkräutern, so z.B. bei Amaranthus hybridus und Solanum nigrum.

Es wäre nun wünschenswert, wenn es gelänge Gene direkt in die Plastide-und Mitochondriengenome von Pflanzen, insbesondere von Kulturpflanzen, einzubauen. Dies würde es ermöglichen, solchen Pflanzen neue, wünschenswerte Eigenschaften zu verleihen. Um Herbizid-resistente Pflanzen zu erzeugen werden beispielsweise Gene, die eine Herbizid-Resistenz vermitteln, in Herbizid-sensitiven Chloroplasten benötigt.

Die bisherigen Anstrengungen galten im allgemeinen einer genetischen Manipulation des nuclearen Genoms der pflanzlichen Zelle, um eine Resistenz oder eine Toleranz gegenüber Herbiziden zu erzielen, die in Chloroplasten aktiv sind.

Diese Vorgehensweise führt jedoch lediglich zu marginalen Toleranzerscheinungen gegenüber diesen Herbiziden, nicht aber zu wirklichen Resistenzen.

Bisher wurde es für unmöglich gehalten, eine echte Resistenz gegenüber Herbiziden, die in Chloroplasten wirksam sind, mit Hilfe des direkten Gentransfers auf Kultur-Pflanzen zu übertragen, da man bisher davon ausging, dass eine Transformation von Plastiden, wie z.B. den Chloroplasten, mit dieser Methode nicht möglich ist.

Ein weiterer Nachteil bei der Einschleusung von Genen, die eine wünschenswerte Eigenschaft, wie z.B. eine Herbizid-resistenz, vermitteln, ins nucleare Genom einer Pflanze liegt in der Fähigkeit einiger Pflanzen zur Fremdbestäubung von Unkräutern begründet. Solche Kreuzungen eröffnen eine Möglichkeit, diese bei Kulturpflanzen erwünschten Eigenschaften in Unkräuter zu übertragen.

Eine der am häufigsten verwendeten Methoden zur Einschleusung von Genen ins nucleare Genom von Pflanzen besteht in der Infektion von Zellen mit Pathogenen, wie z.B. einem Agrobacterium, das Ti-Plasmid Vektor-Systeme enthält. (Barton et al., 1983; Chilton et al., 1985). Diese Verfahren haben aber deutliche Nachteile, die im Zusammenhang mit dem Infektionsvorgang stehen. Diese Nachteile bestehen zum einen in einer begrenzten Wirtspezifität zum anderen in der Notwendigkeit, die transformierten Pflanzenzellen von dem für die Transformation verwendeten Pathogen wieder zu befreien.

Es wurden darüberhinaus Bedenken gegen eine Entlassung solcher Pathogene in die Umwelt laut. (Roberts, 1985).

De Block et al. (1985) berichten über die Verwendung eines Agrobakterium Ti-Plasmid Vektor-Systems für die Einschleusung eines für eine Antibiotika-Resistenz kodierenden Gens, ins Chloroplasten-Genom von Tabak-Protoplasten, aus denen vollständige Zellen und schliesslich komplette, fertile Pflanzen regeneriert werden konnten. Die Autoren fanden jedoch, dass das Gen in Abwesenheit der entsprechenden Antibiotika instabil war und nach kurzer Zeit wieder verloren ging. Die Erhaltung von Pflanzen unter Antibiotika-Selektionsdruck stellt aber keine praktische Anwendung dar.

Verfahren für die direkte Transformation von pflanzlichen Protoplasten mit nackter linearer DNA oder zirkulärer Plasmid-DNA sind ebenfalls bekannt (Paszkowski et al., 1984; sowie Schilperoort et al., 1983). Bei diesen Verfahren wird kein Pathogen für den Infektionsvorgang benötigt. Bis zum jetzigen Zeitpunkt blieben diese Methoden jedoch auf die nucleare Transformation beschränkt.

Es werden daher Verfahren benötigt, die den direkten Transfer von nützlichen Genen in das Genom von Plastiden und Mitochondrien erlauben, so dass vorteilhafte, neue Eigenschaften auf pflanzliche Zellen übertragen werden können, ohne dass eine vorherige Infektion der Zellen mit einem Pathogen notwendig ist und wobei gleichzeitig verhindert wird, dass diese Eigenschaften auf Unkräuter übertragen werden. Weiterhin besteht ein Bedürfnis für ein Verfahren, das den stabilen Transfer von Genen in Plastide und Mitochondrien von Pflanzen-Zellen und ganzen Pflanzen gewährleistet, so dass die Expression besagter Gene nicht während der Entwicklung der Kultur-Pflanzen auf dem Feld verloren geht.

Ein wesentlicher Bestandteil der vorliegenden Erfindung besteht somit in der Bereitstellung eines Verfahrens für die direkte Einführung von Genen in das Genom von Plastiden und Mitochondrien, vorzugsweise von Chloroplasten, ohne Infektion der Zellen mit einem Pathogen und Erhaltung der Gene in besagtem Genom. Ein weiterer Gegenstand der vorliegenden Erfindung besteht in der Herstellung ganzer Pflanzen, die genetisch manipulierte Plastide und Mitochondrien enthalten, und zwar unter Bedingungen, unter denen die mittels rekombinanter Gentechnologie massgeschneiderten Eigenschaft(en) stabil erhalten bleiben und zur Expression gelangen.

Wie aus der nachfolgenden Beschreibung hervorgeht, werden diese und noch weitere Ziele durch die Bereitstellung des erfindungsgemässen Verfahrens zur direkten Einschleusung von DNA in die Plastide und Mitochondiren von pflanzlichen Protoplasten erreicht, wobei besagte DNA aus einem oder mehreren Genen und in Plastiden und Mitochondrien aktiven Promotoren besteht und das Verfahren sich dadurch kennzeichnet, dass man in Abwesenheit eines Pathogens diese besagte DNA mit Protoplasten in einem Medium so lange in Kontakt bringt, dass die Penetration des Gens in die Protoplasten und die darin befindlichen Plastide und Mitochondrien gewährleistet ist.

Abbildungen

Abb. 1 zeigt ein Fliessdiagramm der Einzelschritte für die Konstruktion der Plasmide pCAT⁰ und p32CAT.

Abb. 2 zeigt ein Fliessdiagramm der Einzelschritte für die Konstruktion des Plasmids pUCH1.

Abb. 3 zeigt ein Fliessdiagramm der Einzelschritte für die Konstruktion des Plasmids pBRCAT>

In den beiliegenden Abbildungen werden folgende Kurzsymbole verwendet:
X = XhoI
S = SmaI
H = HindIII
B = BamHI
RI = EcoRI
SI = SalI
LIG. = Ligierung mittels einer T4 DNA Ligase

X/SI bezeichnet eine Stelle, an der durch das Verbinden einer XhoI-Sequenz mit einer SalI-Sequenz eine hybride Restriktionsschnittstelle geschaffen wird, die von keinem der Enzyme geschnitten werden kann.

CAT bezeichnet die kodierende Region der Chloramphenicol-Acetyl-Transferase. CAT⁰ bezeichnet die promotorfreie Form des CAT-Gens.

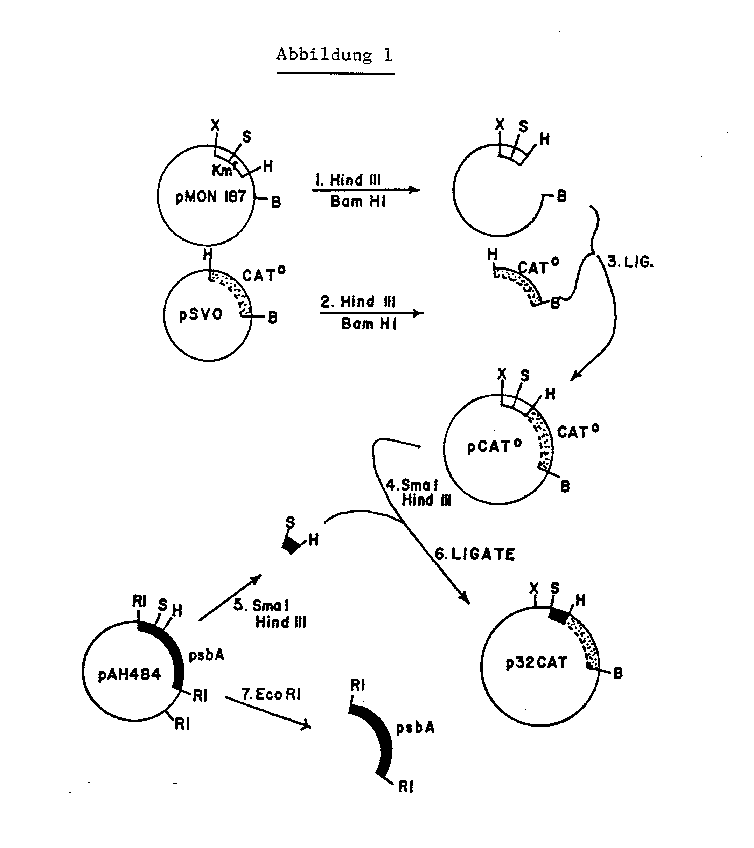

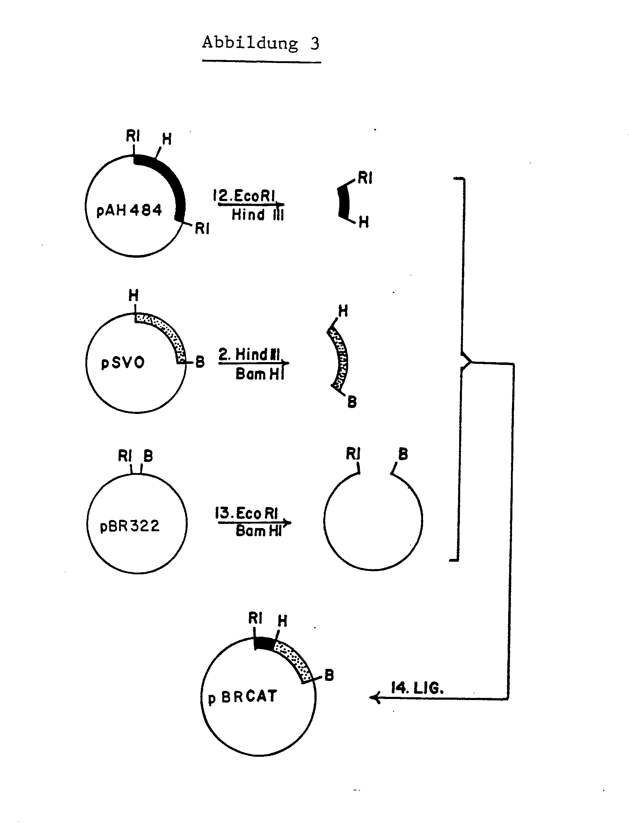

In den Abbildungen 1 bis 3 wird das psbA-Gen durch einen schwarzen Balken, der zugehörige Promotor durch einen schwarzen Kasten charakterisiert.

In den Abbildungen 1 bis 3 ist für CAT kodierende Region punktiert gezeichnet.

In der vorliegenden Erfindungs-Beschreibung und den Patentansprüchen gelten folgende Definitionen:

Ein "Gen" ist eine DNA-Sequenz, gekennzeichnet durch einen Promotor und eine transkribierbare DNA-Sequenz.

Der Promoter, der in den meisten Fällen nicht transkribiert wird, veranlasst die Transkription der nachfolgenden, in einigen Fällen auch den ihn umgebenden Gen-Sequenzen in die entsprechende RNA. Die RNA kann oder auch nicht in ein Polypetid übersetzt werden. Falls die RNA übersetzt wird bezeichnet man sie als mRNA. Die RNA-Sequenz, die der mRNA entspricht sowie auch die mRNA selbst sind aus einer 5' Region, die nicht übersetzt wird, einer kodierenden Region, sowie einer 3' Region zusammengesetzt, die ebenfalls nicht übersetzt wird. Nur die kodierende Region wird in das entsprechende Polypeptid übersetzt.

Die "aktiven" Teile einer DNA-Sequenz bilden diejenigen Abschnitte, die für die Funktion der DNA-Sequenz verantwortlich sind. Einige Beispiele von aktiven Bereichen von DNA-Sequenzen umfassen die RNA-Polymerase-Bindungsstelle, das Initiationssignal (TATA-Box) des Promotors, die Ribosomenbindungsstelle sowie das Translationsinitiationssignal der nicht übersetzten 5'-Region, die kodierende Sequenz, das Transkriptions-Stop-Signal sowie das Polyadenylations-Signal der nicht übersetzten 3'-Region. Die verschiedenen Spacer-Abschnitte, in denen die DNA-Sequenz keine Bedeutung hat, werden nicht als aktive DNA-Sequenzen betrachtet.

Eine "Variante" einer natürlichen DNA-Sequenz bildet eine modifizierte Form der natürlichen Sequenz, die die gleiche Funktion erfüllt. Dabei kann es sich um eine Mutante oder eine synthetische DNA-Sequenz handeln, die im wesentlichen zu der entsprechenden natürlichen Sequenz homolog ist.

Als im wesentlichen homolog zu einer zweiten DNA-Sequenz betrachtet man eine DNA-Sequenz dann, wenn mindestens 70 %, vorzugsweise mindestens 80 %, insbesondere mindestens 90 % der aktiven Anteile der DNA-Sequenz homolog sind. Zur Feststellung der substantiellen Homologie werden zwei verschiedene Nucleotide innerhalb einer DNA-Sequenz einer kodierenden Region immer noch als homolog angesehen, wenn der gegenseitige Austausch dieser Nucleotide zu einer stillen Mutation führt.

Eine DNA-Sequenz "stammt ab von" einer Quelle, wie z.B. den Plastiden oder Mitochondrien, wenn diese DNA-Sequenz in dieser Quelle vorkommt. Die vorliegende Erfindung umfasst auch Varianten einer solchen DNA-Sequenz.

Eine DNA-Sequenz ist "funktionell" in Plastiden oder Mitochondrien, wenn sie ihre erwartete Funktion erfüllt und in den Nachkommen der Plastide und Mitochondrien erhalten bleibt.

Unter "transformierbaren, pflanzlichen Protoplasten", versteht man Protoplasten, die nach einem direkten Gen-Transfer ein Plastid-oder ein Mitochondriengenom enthalten, das kovalent-verknüpfte DNA aufweist, die normalerweise in diesen Plastiden oder Mitochondrien nicht vorkommt.

Pflanzliche Protoplasten die mit Hilfe des erfindungsgemässen Verfahrens transformierbar sind, können von kultivierten Zellen, sowie von Zellen die in Pflanzenteilen oder vielzelligen Pflanzen eingebaut vorliegen; abstammen. Die transformierbaren pflanzlichen Protoplasten können aber ebenso von einzelligen Pflanzen, wie z.B. Algen abstammen. Einige Beispiele einzelliger Pflanzen umfassen die Cyanobacterien (e.g. Synechococcus spp.) sowie Chlamydomonas und Euglena. Besonders bevorzugt im Rahmen der vorliegenden Erfindung sind jedoch pflanzliche Protoplasten, die von vielzelligen Pflanzen abstammen. Nach der erfolgten Transformation können die Protoplasten gegebenenfalls zu ganzen Pflanzen regeneriert werden.

Mit Hilfe der vorliegenden Erfindung ist es nunmehr möglich die Plastide und Mitochondrien jeglicher pflanzlicher Protoplasten gezielt genetisch zu modifizieren.

Einige Beispiele derartiger pflanzlicher Protonplasten sind: Solanum spp. (Kartoffel), Gossypium spp. (Baumwolle), Glycine spp. (Sojabohne), Petunia spp. (Petunie), Daucus spp. (Karotte) Citrus spp (Orange, Zitrone), Lycopersicon spp. (Tomate), Brassica spp. (Rübe, Kohl, Blumenkohl etc), Beta spp. (Rübe), Phaseolus spp. (Bohne), Helianthus spp. (Sonnenblume), Arachis spp. (Erdnuss), Amaranthus spp. (Fuchsschwanz), Medicago spp. - (Alfalfa), Trifolium spp. (Klee), Atropy spp. (Nachtschatten), Hyoscyamus spp. (Bilsenkraut), Digitalis spp. (Fingerhut), Catharanthus spp. (Immergrün), Pisum spp. (Erbse), und Pinus spp. (Kiefer).

Transformierbare Plastide sind z.B. Chromoplasten, Amyloplasten, Leucoplasten, Etioplasten, Proplastide und Chloroplasten. Besonders bevorzugt für die Transformation sind die Chloroplasten.

Die vorliegende Erfindung betrifft ferner Gene, die in den Plastiden und Mitochondrien funktionsfähig sind. Die Gene der vorliegenden Erfindung übertragen durch ihre Einschliessung auf die Plastide und Mitochondrien eine gewünschte, nützliche Eigenschaft. Einige Beispiele für solche nützlichen Eigenschaften sind: die Phytotoxin-Resistenz, wie z.B. Herbizid-oder Antibiotika-Resistenz, verbesserte photosynthetische Effizienz sowie Enzymaktivität in Fällen, in denen ein chromogenes Substrat für das Enzym bekannt ist.

Unter Herbizid-Resistenz versteht man im vorliegenden Fall eine Resistenz gegenüber allen Herbiziden, die in den Plastiden oder Mitochondrien aktiv sind. So sind beispielsweise zahlreiche Herbizide in Chloroplasten aktiv. Zu diesen Herbizidklassen gehören z.B. die Triazin-, Harnstoff-, Sonfonylharnstoff-sowie die Uracilderivate, ebenso wie das Glyphosate (N-Phosphomethylglycin).

Einige Beispiele für Triazin-Herbizide umfassen das Atrazin, Ametryn, Metribuzin sowie das Simazin. Einige Beispiele für Harnstoff-Herbizide - schliessen die Phenylharnstoffe, wie z.B. Diuron, Chloroxuron und Fluormeturon sowie das DCMU (Dichlormethylharnstoff) ein. Beispiele für Sulfonylharnstoffe sind Oust and Glean, sowie für Uracil-Herbizide das Bromacil und Terbacil. Diese und andere Herbicide sind in LeBaron und Gressel - (1982) beschrieben.

Um eine Zelle gegenüber Herbiziden resistent zu machen, ist es nicht nötig, dass man mittels direktem Gentransfer in jeden der 50 bis 100 Chloropasten dieser Zelle ein für die Herbizid-Resistenz kodierendes Gen einschleust. Ein direkter Gentransfer in einen kleinen Teil der vorhandenen Chlorodplasten ist völlig ausreichend um die Zellen vor Herbiziden zu schützen.

Die Möglichkeit eine Herbizid-Resistenz z.B. gegen Atrazin, auf Pflanzen zu übertragen ist aus verschiedenen Gründen wünschenswert. Sie erlaubt beispielsweise die Anwendung dieses Herbizids in höheren Dosen auf Pflanzen, die dann tolerant sind gegenüber besagtem Herbizid. Mit den höheren Herbizid-Dosen erzielt man dann gleichzeitig eine bessere Effizienz in der Unkrautbekämpfung.

Darüberhinaus kann die Herbizid-Resistenz aber auch als selektiver Marker verwendet werden, der genetisch mit einer physiologischen Eigenschaft gekoppelt wird, die an sich schwierig zu selektieren ist. Um diese Möglichkeit nutzen zu können, wird ein Donor-Plasmid konstruiert, das ein Gen enthält, das eine Herbizid-Resistenz bewirkt sowie ein zweites Gen, das die andere wünschenswerte Eigenschaft vermittelt. Dabei kann es sich beispielsweise um eine erhöhte photosynthetische Effizienz handeln. Diese Donor-DNA wird in pflanzliche Protoplasten eingeschleust, die zu einer pflanzlichen Zellkultur oder zu ganzen Pflanzen regeneriert werden können. Die resultierenden Pflanzen besitzen dann sowohl die Eigenschaften der Herbizid-Resistenz aus auch die besagte zweite Eigenschaft. Lässt man diese Pflanzen in Gegenwart des entsprechenden Herbizids wachsen, so ist es möglich, Pflanzen zu selektionieren, die diese besagte zweite Eigenschaft besitzen.

Darüberhinaus gestattet die Einführung einer Donor-DNA, die sowohl eine Herbizid-Resistenz, als auch eine zweite aus angronomischer Sicht nützliche Eigenschaft auf pflanzliche Chloroplasten überträgt, diese zweite Eigenschaft in den Chloroplasten stabil zu erhalten, wenn man die Pflanzen in Gegenwart des Herbizids anbaut.

Auf die Instabilität fremder Gene in Chloroplasten hat bereits De Block et al. (1985) hingewiesen (siehe oben).

Besonders bevorzugt ist eine Herbizid-Resistenz gegenüber Atrazin (2-Chlor-4-ethylamino-6-isopropylamino-1,3,5-triazin).

Für die in vitro-Identifizierung von Zellen mit transformierten Plastiden oder Mitochondrien, kann eine Antibiotika-Resistenz verwendet werden.

Eine Antibiotika-Resistenz ist eine Eigenschaft, die für die in vitro-Identifizierung von Zellen, die transformierte Plastide oder Mitochondrien enthalten, verwendet werden kann. Gene, die diesen selektierbaren Marker tragen, sind ausserordentlich nützlich, wenn sie auf genetischem Weg mit agronomisch nützlichen Eigenschaften verknüpft werden. Hierfür eignen sich beispielsweise Resistenzen gegen Chloramphenicol, Kanamycin oder aber in Prinzip auch Resistenzen gegenüber beliebigden anderen Antibiotika.

Eine nützliche Eigenschaft, die in erster Linie als Marker für eine Auslese (Screening) zur Identifizierung von Pflanzen-Zellen von Gewebe-Kulturen geeignet ist, die genetisch manipulierte Plastide oder Mitochondrien enthalten, erreicht man z.B. durch Einschleusung eines Gens, das für ein Enzym kodiert, welches ein farbgebendes Substrat besitzt. Handelt es sich bei besagtem Enzym beispielsweise um beta-Galaktosidase, so werden die Pflanzen-Zellen auf einem Gewebekultur-Medium ausplatiert, das das farbgebende Substrat Xgal (5-Chlor-4-brom-3-indolyl-$\beta$-D-galactosid) enthält. Pflanzenzellen, die genetisch manipulierte Plastide oder Mitochondrien enthalten, werden dabei durch den Farbstoff Indigoblau angefärbt, da dieser aufgrund der Spaltung von Xgal durch $\beta$-Galaktosidase freigesetzt wird.

Die Gene, die für die vorliegende Erfindung geeignet sind, können nach an sich bekannten Methoden gewonnen werden. Bei diesen Methoden handelt es sich z.B. um die Isolierung natürlicher, normalerweise nur ausserhalb der Plastide oder Mitochondrien vorkommender Gene oder deren Varianten, die eingeschleust werden sollen.

Besagtes Gen kann jedes natürlich vorkommende Gen oder eine seiner in den Plastiden oder Mitochondrien funktionsfähigen Varianten sein. Bevorzugt sind natürlich vorkommende Platid-, Mitochondrien-oder Bakterien-Gene. Besonders bevorzugt sind Chloroplast-Gene.

Um sicher zu sein, dass sich besagtes Gen tatsächlich in den Plastiden oder Mitochondrien befindet und nicht etwa im Zellkern, kann man vorteilhafterweise, aber nicht zwingend notwendig, ein im Zellkern nicht oder aber zumindest deutlich weniger als in den Platiden oder Mitochondrien funktionsfähiges Gen einsetzen.

Einige Beispiele natürlich vorkommender Bakterien-Gene sind z.B. solche, die für eine Antibiotika-Resistenz oder für Enzyme mit einem farbgebenden Substrat kodieren. Ein bakterielles Gen, das in der Lage ist eine Antibiotika-Resistenz auf Plastide oder Mitochondrien zu übertragen, ist z.B. das Chloramphenicol-Acetyl-Transferase-Gen. Ein bakterielles Gen, das für ein Enzym mit chromogenem Substrat codiert, ist z.B. lacZ, das für die $\beta$-Galaktosidase kodiert. Einige Beispiele von Plastid-oder Mitochondrien-Genen sind Gene, die für eine Herbizid-Resistenz oder für eine verbesserte photosynthetische Effizienz kodieren. Ein Chlorplasten-Gen, das in der Lage ist eine Herbizid-Resistenz zu übertragen, ist z.B. das mutierte psbA Gen, welches von Hirschberg et al. (1983) beschrieben wird oder eine Mutante des psbD-Gens (Rochaix et al., 1984). Ein Chloroplasten-Gen, das in der Lage ist eine verbesserte photosynthetische Effizienz zu übertragen, wird z.B. durch eine modifizierte Form von rbcL repräsentiert, das für eine modifizierte grosse Untereinheit der Ribulose-1,5-bisphosphatcarboxylase/oxygenase (Rubisco) kodiert. Ein Gen, das die grosse Untereinheit der Rubisco exprimiert, ist bereits in den Chloroplasten enthalten und bei der Photosynthese beteiligt. Es ist aber auch möglich, eine modifizierte (mutierte, manipulierte oder heterologe) Form diese Gens mit verbesserter photosynthetischer Effizienz [Jordan et al., (1981)] einzuschleusen. Eine weitere Möglichkeit ein Gen zu erhalten, das in den Plastiden oder Mitochondrien funktionsfähig ist, besteht in der Konstruktion eines chimären Gens. Ein chimäres Gen ist ein Gen oder zumindest ein Teil eines Gens, vorzugsweise ein aktiver Teil eines Gens, der natürlicherweise nicht kovalent an die übrigen Teile gebunden ist. Das chimäre Gen kann entweder ein RNA-Transkript spezifizieren oder für ein Polypeptid kodieren.

Als Promotoren des chimären Gens kommen in Rahmen der vorliegenden Erfindung alle Promotoren in Frage, die in Plastiden oder Mitochondrien funktionsfähig sind. Promotoren für chimäre Gene können von einem natürlich vorkommenden Plastid-oder Mitochondrien-Gen oder von einem Gen stammen, das nicht in den Plastiden oder Mitochondrien vorkommt. Unter den natürlich vorkommenden Promotoren aus Plastiden oder Mitochondrien-Genen sind insbesondere solche bevorzugt, die aus psbA, psbD und rbcL-Genen stammen. Bei besagten Promotoren kann es sich ebenso um Varianten natürlicher Promotoren handeln. Bei einem heterologen Promotor des chimären Gens kann es sich erfindugnsgemäss um einen natürlichen Promotor handeln, der normalerweise nicht in Plastiden oder Mitochondrien vorkommt.

Da die Funktionen von Plastid-oder Mitochondrien-Genen oft ähnlich oder mit den Fuktionen von bakteriellen Genen identisch sind, können auch bakterielle Promotoren in Plastiden oder Mitochondrien funktionsfähig sein. Jeder bakterielle Promotor, der in Plastiden oder Mitochondrien funktionsfähig ist, kann daher auch erfindungsgemäss als Promotor des chimären Gens eingesetzt werden. Einige brauchbare bakterielle Promotoren sind z.B. solche des Neomycin-Phosphotransferase-II-Gens und der T-DNA-Gene, wie z.B. das Nopalin-Synthase-Gen des Ti-Plasmids.

Als heterologer Promotor kommt auch jeder teilweise oder vollständig synthetisch hergestellte Promotor in Frage, der in Plastiden oder Mitochondrien funktionsfähig ist.

Teilweise oder vollständig synthetisch hergestellte Promotoren, die ihren natürlichen Vorbildern dadurch ähneln, dass sie 10 Nucleotide unterhalb des Transkriptions-Starks eine TATAAT-ähnliche Sequenz aufweisen, sind ebenfalls Bestandteil vorliegender Erfindung.

Bei der nicht übersetzten 5′-Region des chimären Gens der vorliegenden Erfindung kann es sich um jede beliebige nicht übersetzte 5′-Region handeln, die in den Plastiden oder Mitochondrien funktionsfähig ist. Die nicht übersetzte 5′-Region kann beispielsweise von Plastid-oder Mitochondrien-Genen oder von Genen anderen Ursprungs stammen. Eine bevorzugte homologe nicht übersetzte 5′-Region stammt von den psbA, psbD oder rbcL-Genen. Eine bevorzugte heterologe nicht übersetzte 5′-Region stammt von bakteriellen Genen. Synthetische nicht übersetzte 5′-Regionen, die aufgrund einer effektiven Ribosomenbindungsstelle ihren natürlichen Vorbildern ähneln, sind ebenfalls Bestandteil vorliegender Erfindung.

Im Prinzip eignet sich jede beliebige kodierende Region, die in der Lage ist eine wünschenswerte Eigenschaft auf eine Pflanzen-Zelle zu übertragen, für die erfindungsgemässe Verwendung als kodierende Region. Sowohl die kodierenden Regionen der oben erwähnten natürlicherweise vorkommenden Gene als auch kodierende Regionen aus anderen Quellen können im Rahmen der vorliegenden Erfindung für die Herstellung eines chimären Gens verwendet werden.

Folglich können die kodierenden Regionen im Rahmen der vorliegenden Erfindung von natürlicherweise vorkommenden Plastid-oder Mitochondrien-Genen oder von einer anderen Quelle stammen; sie können auch vollständig oder teilweise synthetisch oder durch Fusion zweier oder mehrerer solcher kodierender Regionen unter Beibehaltung des Leserahmens hergestellt sein.

Jede dieser kodierenden Abschnitte kodiert für ein Polypeptid, das eine oder mehrere neue Eigenschaften auf die Zellen und Pflanzen überträgt.

Ein Beispiel für ein Polypeptid, das durch ein natürlicherweise in den Chloroplasten bestimmter Pflanzen vorkommendes Gen bestimmt wird, ist die mutierte Form des 32 kd Polypeptids, das von Hirschberg et al. (1983) beschrieben wird. Es konnte gezeigt werden, dass dieses Polypeptid eine Atrazin-Resistenz auf bestimmte Unkräuter überträgt. Das psbA-Gen, welches für besagtes Polypeptid kodiert, kann aber auch auf nützliche Pflanzen, wie z.B. Kulturpflanzen unter Verwendung des erfindungsgemässen Verfahrens übertragen werden.

Ein Beispiel für eine kodierte Region, die nicht aus Plastiden oder Mitochondrien stammt, ist die kodierende Region der pflanzlichen, tierischen oder bakteriellen gshA und gshB-Gene oder die des Glutathion-Reduktase-Gens (gor), wobei alle ihre nützlichen Eigenschaften übertragen können, wenn sie in pflanzliche Plastiden oder Mitochondrien eingeschleust werden. gshA und gshB kodieren Enzyme, die die Synthese des Tripeptides Glutathion katalysieren, welches seinerseits für die konjugative Detoxifikation zahlreicher Herbizide verantwortlich ist (Meister et al., 1983; sowie Rennenberg; 1982).

Ein weiteres Beispiel für eine kodierende Region, die nicht aus Plastiden oder Mitochondrien stammt, ist der kodierende Abschnitt eines Glutathion-S-Transferase-Gens aus Pflanzen, Tieren, Insekten oder Bakterien. In manchen Pflanzen [Shimabukuroy et al., (1971)], die bekanntermassen gegenüber dem Herbizid Atrazin tolerant sind, bildet die Anwesenheit der Glutathion-S-Transferase-

(n) die Grundlage dieser Toleranz. Die Glutathion-S-Transferase detoxifiziert das Phytotoxin, indem es die Bildung eines Atrazin-Glutathion-Konjugates katalysiert.

Eine andere kodierende Region, die für das chimäre Gen der vorliegenden Erfindung brauchbar ist, kodiert eine effizientere Form von Rubisco. Solche kodierenden Abschnitte können gegebenenfalls von natürlicherweise vorkommenden Genen stammen.

Die kodierende Region der vorliegenden Erfindung kann gegebenenfalls auch aus zwei oder mehreren verschieden kodierenden Abschnitten herrühren. Polypeptide, die durch solche kodierenden Regionen spezifiziert werden bezeichnet man als Fusions-Polypeptide.

Ein Beispiel für eine Fusions-Polypeptid stellt eine effizientere Form von Rubisco dar. Rubisco hat zwei Funktionen, zum einen als Carboxylase zum anderen als Oxygenase. Die Carboxylase-Funktion ist im Verlauf der Photosynthese wirksam, die Oxygenase-Funktion dagegen ist unerwünscht. Ein brauchbares Fusions-Polypeptid besitzt dementsprechend einen Anteil der die Carboxylase-Funktion maximiert und einen zweiten Teil, der die Oxygenase-Funktion minimiert.

Die nicht translatierte 3'-Region des chimären Gens kann entweder natürlicherweise in einem Plastid-oder Mitochondrien-Gen vorliegen oder sie kann heterologen Ursprungs sein. Bevorzugt ist eine nicht translatierte 3'-Region, die aus einem Plastid-oder Mitochondrien-Gen stammt. Die bevorzugten nicht translatierten 3'-Abschnitte sind diejenigen der psbA, psbD oder rbcL-Gene.

Die transkribierten Regionen der Gene der vorliegenden Erfindung können RNA-Transkripte spezifizieren und sind in einigen Fällen schon als solche verwendbar.

Bei diesen Transkripten kann es sich um tRNA oder rRNA handeln. Das Transkript kann ebenso eine RNA sein, die eine Sequenz besitzt die zumindest einem Teil einer Sequenz eines anderen RNA-Transkripts komplementär ist. Solche komplementären Sequenzen, die unter der Bezeichnung Anti-sense Sequenzen bekannt sind, können, falls sie nur lang genug sind, die Funktion von RNA-Sequenzen, zu denen sie komplementär sind, stören oder sie können die Transkription dieser RNA von dem entsprechenden Gen blockieren; [Vgl. Pestka et al., (1984); Melton, (1985); Izant et al., (1984); Simons et al., (1984) und Coleman, -(1984)].

Transkribierte Regionen, die im Rahmen der vorliegenden Erfindung brauchbar sind, können gegebenenfalls von natürlicherweise transkribierten Abschnitten stammen oder aber sie können ganz oder teilweise synthetisch hergestellt sein. Anti-sense-Sequenzen können ebenso von zumindest einem Teil eines natürlicherweise vorkommenden Gens stammen, indem man die Orientierung besagten Teils des natürlicherweise vorkommenden Gens in Bezug auf seinen Promotor umkehrt.

Die Gene, die für eine Verwendung im Rahmen der vorliegenden Erfindung geeignet sind, werden nach an sich bekannten Methoden in ein Plasmid-Klonierungs-Vektor eingebaut bzw. eingeführt - [Maniatis et al., (1982)].

Für die Integration von Fremdgenen in die genomische DNA von Pflanzen-Zellen ist es vorteilhaft, wenn die Gene von neutralen DNA-Sequenzen, sogenannter Carrier-DNA, flankiert werden. Die Carrier-DNA kann aus zwei linearen DNA-Strängen bestehen, so dass die gesamte Konstruktion, die in die Pflanzenzelle eingeschleust werden soll, ein lineares DNA-Molekül darstellt. Besagtes Gebilde kann für die Gen-Transformation aber ebenso eine zirkuläre Struktur -Plasmid-Struktur - aufweisen. Die Carrier-DNA kann sowohl synthetischen Ursprungs sein als auch von natürlicherweise vorkommenden DNA-Sequenzen abstammen, die mit geeigneten Restriktionsendonukleasen behandelt wurden. Daher sind beispielsweise auch natürlich vorkommende Plasmide, die mit ein oder mehreren Restriktionsendonukleasen geöffnet wurden für die Verwendung als Carrier-DNA geeignet. Als Beispiel für ein solches Plasmid ist das leicht zugängliche Plasmid pUC8 [beschrieben bei Messing et al., (1982)] zu nennen. Fragmente von natürlicherweise vorkommenden Plasmiden können ebenso als Carrier-DNA erfindungsgemäss verwendet werden. Die für die Transformation vorgesehene Konstruktion kann gegebenenfalls von einem Ti-Plasmid oder von einem modifizierten Ti-Plasmid stammende DNA enthalten. Ein Plasmid wird als modifiziertes Ti-Plasmid angesehen, wenn es zumindest ein T-DNA-Grenzregion besitzt. Eine T-DNA Grenzregion ist eine DNA-Sequenz innerhalb eines Agrobakterium-Genoms, welche den Einbau einer anderen Sequenz innerhalb des Genoms (T-DNA) in die Pflanzenzellen mit denen das Agrobacterium in Kontakt gerät verursacht. Bei der T-DNA Grenzregion kann es sich beispielsweise um eine Sequenz eines Vektors handeln, wie z.B. eines Ti-oder Ri-Plasmids oder eines modifizierten Ti-oder Ri-Plasmids. Bei der T-DNA kann es sich auch um eine Sequenz handeln, die auf dem selben Vektor liegt wie die Grenz-Region oder aber auf einem anderen Vektor.

Die Wahrscheinlichkeit der genetischen Transformation (Transformationsrate) einer Pflanzenzelle kann durch verschiedene Faktoren gesteigert werden. Wie aus Experimenten mit Hefe bekannt ist, steigt dementsprechend die Anzahl geglückter stabiler Gen-Transformationen:

1) mit der Zunahme der Zahl der Kopien von neuen Genen pro Zelle,

2) wenn ein Replikationssignal mit einem neuen Gen kombiniert wird und

3) wenn ein Integrationssignal mit einem neuen Gen kombiniert wird, wobei unter einem Integrationssignal ein Signal zu verstehen ist, das die Integration eines DNA-Stranges in einen anderen DNA-Strang fördert.

Das erfindungsgemässe Verfahren findet daher dann eine besonders vorteilhafte Anwendung, wenn das übertragene Gen mit einem Replikationssignal gekoppelt ist, das in pflanzlichen Zellen wirksam ist oder mit einem Integrationssignal, das in pflanzlichen Zellen wirksam ist oder mit einer Kombination beider Signale.

Protoplasten, Zellkultur-Zellen, Zellen in Pflanzengeweben, Pollen, Pollenschläuchen, Eizellen, Embryonalsäcke oder Zygoten sowie Embryonen in unterschiedlichen Entwicklungsstadien sind repräsentative Beispiele für Pflanzenzellen, die als Ausgangsmaterial für eine Transformation geeignet sind. Bevorzugt sind Protoplasten, da diese direkt ohne weitere Vorbehandlung verwendet werden können. Isolierte pflanzliche Protoplasten, Zellen oder Gewebe können mit Hilfe an sich bekannter Methoden oder mit Hilfe von Methoden, die analog zu bekannten Methoden sind, gewonnen werden.

Isolierte pflanzliche Protoplasten, die sich als Ausgangsmaterial für die Gewinnung von isolierten Zellen und Geweben eignen, lassen sich aus allen Teilen der Pflanze, z.B. den Blättern, Embryonen, Stielen, Blüten, Wurzeln oder Pollen isolieren. Bevorzugt werden Protoplasten aus Blättern. Isolierte Protoplasten können aber auch aus Zellkulturen erhalten werden. Methoden zur Isolierung von Protoplasten werden beispielsweise in Gamberg et al. (1975) beschrieben.

Der Transfer des neuen Gens in die Pflanzenzelle erfolgt auf direktem Wege, d.h. ohne vorherige Infektion der Zelle mit einem Pathogen wie beispielsweise einem pflanzenpathogenen Bakterium, Virus oder Pilz und ohne Uebertragung von DNA durch Insekten oder Pilze, die in der Lage sind Pflanzen mit DNA-übertragenden Pathogenen zu infizieren. Dieser direkte Gentransfer wird dadurch erreicht, dass die das Gen enthaltende DNA mit den pflanzlichen Protoplasten und den darin enthaltenen Plastiden und Mitochondrien in einem Medium für eine solche Zeitspanne in Kontakt bringt, welche für die Penetration des Gens in die Protoplasten und in die darin befindlichen Plastide und Mitochondrien ausreicht.

Die Transformationsfrequenz kann dadurch erhöht werden, dass man diesen Schritt mit verschiedenen Gentransfer-Techniken kombiniert. Beispiele solcher Techniken umfassen die Behandlung mit Poly-L-Ornithin oder Poly-L-Lysin, die Liposomenfusion, DNA-Protein-Komplexierung, Veränderung der Ladungsverhältnisse an der Protoplastenmembran, Fusion mit mikrobiellen Protoplasten oder Kalziumphosphat-Präzipitation sowie insbesondere durch Behandlung mit bestimmten polyhydrierten Alkoholen wie z.B. Polyethylenglykol, ferner durch Hitzeschockbehandlung und Elektroporation sowie durch eine Kombination dieser zuletzt genannten drei Techniken.

Als erfindungsgemäss verwendbare Medien kommen alle Medien in Frage, die der DNA, die das Gen trägt, die Penetration in die Protoplasten sowie in die Plastide oder Mitochondrien innerhalb dieser Protoplasten erlauben.

Geeignete Medien für die gemeinsame Inkubation des Fremdgehens mit dem Rezeptor-Protoplasten sind vorzugsweise osmotisch stabilisierte Kulturmedien, wie sie für Protoplasten-Kulturen entwickelt wurden.

Zahlreiche, inzwischen erhältliche Kultur-Medien unterscheiden sich in einzelnen Komponenten oder Gruppen von Komponenten. Die Zusammensetzung all dieser Medien steht jedoch in Einklang mit dem Prinzip, dass sie alle eine Gruppe anorganischer Ionen in einem Konzentrationsbereich von ca. 10 mg/Liter bis zu einigen hundert mg/Liter (sogenannte Makroelemente wie Nitrate, Phosphate, Sulfate, Kalzium, Magnesium, Eisen, etc.) besitzen; eine weitere Gruppe anorganischer Ionen mit einer maximalen Konzentrationen von einigen mg/Liter (sogenannte Spurenelemente wie Kobalt, Zink, Kupfer, Mangan, etc); eine Anzahl von Vitaminen (z.B. Inositol, Folsäure, Thiamin); eine Energie-und Kohlenstoffquelle, beispielsweise Sucrose oder Glucose und Wuchsregulatoren in Form natürlicher oder synthetischer Phytohormone der Auxin-und Cytokinin-Klasse in einem Konzentrationsbereich von 0.01 bis 10 mg/Liter.

Diese Kulturmedien sind zusätzlich noch osmotisch stabilisiert durch Zusatz von Zuckeralkoholen (z.B. Mannitol), Zucker (z.B. Glucose) oder Salz-Ionen (z.B. $CaCl_2$) sowie auf einen pH-Wert im Bereich von 5.6 bis 6.5 eingestellt.

Eine ausführlichere Beschreibung konventioneller Kulturmedien findet man beispielsweise bei Kobliz et al. (1974).

Ein besonders geeignetes Medium für die direkte Transformation von Protoplasten enthält einen mehrwertigen Alkohol, der in der Lage ist die Protoplastenmembran zu verändern und der sich bei der Vermittlung der Zellfusion als nützlich erweist. Der bevorzugte mehrwertige Alkohol ist Polyethylenglykol. Mehrwertige Alkohole mit längeren Ketten, beispielsweise Polypropylenglykol (425 bis 4000 g/mol), Polyvinylalkohol oder mehrwertige Alkohole, deren Hydroxylgruppen teilweise oder komplett alkyliert sind, können auch verwendet werden. Die polyhydroxylierten Detergentien,

die gewöhnlich in der Landwirtschaft Verwendung finden und von den Pflanzen toleriert werden, stellen ebenfalls geeignete mehrwertige Alkohole dar: Solche Detergentien sind z.B. in der folgenden Publikation beschrieben:

"Mc Cutcheons's Detergents and Emulsifiers Annual"
MC Publishing Corp., Ridgewood, New Jersey, - (1981);
Stache, H., "Tensid-Taschenbuch",
Carl Hanser Verlag, München/Wien, (1981).

Die hier bevorzugten mehrwertigen Alkohole sind Polyethylenglykole mit einem Molekulargewicht zwischen 1000 und 10000 g/mol, vorzugsweise zwischen 3000 und 8000 g/mol.

Die Transformationsfrequenz des direkten Gentransfers kann durch die nachfolgend im Detail beschriebenen Verfahren wesentlich erhöht werden.

Bei der Polyethylenglykol Behandlung wird zunächst eine Protoplasten-Suspension dem Kultur-Medium zugegeben. Die DNA, die das Gen enthält und als lineare DNA oder in Form eines zirkulären Plasmids vorliegen kann, wird anschliessend zur vorgelegten Mischung aus Polyethylenglykol und Kultur-Medium gegeben. Alternativ zu dieser Vorgehensweise können auch zuerst die Protoplasten sowie die das Gen enthaltende DNA im Kultur-Medium vorgelegt und dann erst das Polyethylenglykol hinzugefügt werden.

Im Rahmen der vorliegenden Erfindung erwiesen sich die Elektroporation sowie die Hitzeschockbehandlung ebenfalls als besonders vorteilhafte Verfahrensmassnahmen.

Neumann et al. (1982) berichten, dass bei der Elektroporation Protoplasten zunächst in ein Osmotikum überführt werden, z.B. in eine Mannitol/Magnesium-Suspension und diese Protoplastensuspension anschliessend in eine Elektroporator-Kammer zwischen zwei Elektroden eingebracht wird. Durch Entladung eines Kondensators über der Suspension werden die Protoplasten für einen kurzen Moment mit einem elektrischen Impuls von hoher Spannung beaufschlagt, was zu einer Polarisation der Protoplastmembran und einer Oeffnung von Poren in der Membran führt.

Bei der Hitzebehandlung werden die Protoplasten in einem Osmotikum suspendiert, z.B. einer Lösung von Mannitol/Calciumchlorid. Anschliessend wird die Suspension in kleinen Behältern z.B. in Zentrifugenröhrchen, vorzugsweise im Wasserbad, erhitzt. Die Dauer des Erhitzungsvorgangs hängt von der vorgewählten Temperatur ab. Im allgemeinen bewegen sich die Temperaturen im Bereich von 40°C bis 80°C und werden für die Dauer von 1 Sekunde bis zu einer Stunde aufrechterhalten. Die besten Ergebnisse erzielt man bei einer Temperatur im Bereich von 40° bis 50°C bis 4 bis 6 Minuten, insbesondere bei 45° und 5 Minuten. Die Suspension wird anschliessend auf Raumtemperatur oder weniger abgekühlt. Es kann ebenso gezeigt werden, dass die Transformations-Frequenz durch Inaktivierung extrazellulärer Nukleasen gesteigert wird. Die Inaktivierung kann durch Verwendung divalenter Kationen, die von Pflanzen toleriert werden, wie beispielsweise Magnesium oder Kalzium erreicht werden. Die Inaktivierung ist effektiver, wenn die Transformation bei hohen pH-Werten durchgeführt wird, wobei der optimale pH-Bereich zwischen 9 und 10.5 liegt.

Ueberraschenderweise führt die selektive Nutzung dieser verschiedenen Methoden zu einer deutlichen Erhöhung der Transformations-Frequenz, was ein bereits seit langem angestrebtes Ziel auf dem Gebiet des Genetic Engineering darstellt.

Je geringer die Transformations-Frequenz bei den Gen-Transformationsexperimenten ist, um so schwieriger und zeitaufwendiger ist es, die wenigen klonierten Zellen, die von transformierten Zellen abstammen, unter der grossen Zahl von nicht transformierten Klonen herauszufinden. Bei einer nur geringen Transformations-Frequenz ist es praktisch unmöglich, die konventionellen Screening-Methoden anzuwenden, es sei denn, das verwendete Gen besitzt einen selektiven Marker - (z.B. Resistenz gegenüber einer bestimmten Substanz). Geringe Transformations-Frequenzen erfordern daher einen sehr beträchtlichen Arbeits-und Zeitaufwand, wenn man Gene ohne Marker-Funktionen verwendet.

Durch das Zusammenbringen von Fremdgen und Rezeptor-Protoplasten vor der Anwendung anderer Massnahmen, wie z.B. der Polyethylenglykol-Behandlung, der Elektroporation und der Hitzeschockbehandlung, lässt sich, im Vergleich zu einer Vorgehensweise, bei der die einzelnen Verfahrensschritte in einer anderen Reihenfolge durchgeführt werden, eine signifikante Verbesserung in der Transformations-Frequenz erreichen .

Eine Kombination von zwei oder drei der im folgenden aufgezählten Techniken hat sich als vorteilhaft erwiesen: Polyethylenglykolbehandlung, Hitzeschockbehandlung und Elektroporation, wobei besonders gute Ergebnisse erzielt werden, wenn diese Techniken nach dem Einbringen des Fremdgens und der Protoplasten in einer Flüssigkeit angewendet werden. Die bevorzugte Verfahrensweise besteht in einer Hitzeschockbehandlung vor der Poylethylenglykol-Behandlung und einer gegebenenfalls nachfolgenden Elektroporation. Im allgemeinen führt die zusätzliche Elektroporation zu einer weiteren Zunahme der Transformations-Frequenz; in manchen Fällen können die Ergebnisse die durch Hitzeschockbehandlung und Polyäthylenglykolbehandlung erzielt wurden jedoch auch durch eine zusätzliche Elektroporatioin nicht wesentlich verbessert werden.

Man kann ferner auch divalente Kationen einsetzen, die von Pflanzen toleriert werden und/oder eine Transformation bei pH-Werten zwischen pH 9 und 10.5 mit den einzelnen, die Transformationsfrequenz verändernden, oben bereits beschriebenen Massnahmen kombinieren, nämlich mit der Polyethylenglykol-Behandlung, der Hitzeschockbehandlung und der Elektroporation.

Das erfindungsgemässe Verfahren macht es somit möglich, hohe Transformations-Frequenzen zu erreichen ohne Pathogene wie z.B. Viren und Agrobakterium oder natürliche oder modifizierte Ti-Plasmid für die Transformation verwenden zu müssen.

Eine vorteilhafte Ausführungsform des erfindungsgemässen Verfahren ist beispielsweise dadurch charakterisiert, dass die Protoplasten in eine Mannitol-Lösung überführt werden und anschliessend die so erhaltene Protoplasten-Suspension mit einer wässrigen DNA-Lösung, die das Gen enthält, vermischt wird. Die Protoplasten werden dann in diesem Gemisch 5 Minuten bei 45°C inkubiert und anschliessend 10 Sekunden auf 0°C abgekühlt. Im Anschluss an die Inkubation wird diesem Gemisch so viel Polyethylenglykol (MG 3000 bis 8000) zugesetzt, dass eine PEG-Konzentration von 1 bis 25 %, vorzugsweise um 8 %, erreicht ist. Nach vorsichtigem und sorgfältigem Vermischen erfolgt dann die Behandlung in dem Elektroporator. Die Protoplastensuspension wird sodann mit Kulturmedium verdünnt, und kann - schliesslich zur Regeneration ihrer Zellwände im Kulturmedium verbleiben.

Das erfindungsgemässe Verfahren ist für die Transformation aller pflanzlichen Zellen geeignet, insbesondere für Zellen aus den systematischen Gruppen der Angiospermae und Gymnospermae.

Unter den Gymnosperae sind in erster Linie Pflanzen aus der Klasse Coniferae von Interesse.

Unter den Angiospermae sind neben Laubbäumen und Sträuchern Pflanzen aus den folgenden Familien von besonderem Interesse: Solanaceae, Cruciferae, Compositae, Liliaceae, Vitaceae, Chenopodiaceae, Rutaceae, Bromeliaceae, Rubiaceae, Theaceae, Musaceae oder Gramineae und innerhalb der Ordnung Leguminosae die Familie der Papilionaceae.

Besonders bevorzugt sind Vertreter aus den Familien der Solanaceae, Cruciferae und Gramineae.

Von besonderem Interesse sind Pflanzen aus der Gattung Nicotiana, Petunia, Hyoscyamus, Brassica und Lolium, so zum Beispiel Nicotiana tabacum, Nicotiana plumbaginifolia, Petunia hybrida, Hyoscyamus muticus, Brassica napus, Brassica rapa und Lolium multiflorum.

Plastide und Mitochondrien all jener Pflanzen, die aus Protoplasten regenerierbar sind, können ebenfalls erfolgreich mit Hilfe des erfindungsgemässen Verfahrens tranformiert werden. Bis zum heutigen Zeitpunkt war es nicht möglich, Plastide und Mitochondrien von Vertretern aus der Familie der Graminae (Gräser) die ebenso Getreidearten, wie z.B. Mais, Weizen, Reis, Gerste, Hafer, Hirse, Roggen und Sorghum umfassen, genetisch zu manipulieren. Die vorliegende Erfindung erlaubt nunmehr die genetische Transformation von Plastiden und Mitochondrien von Gramineen-Zellen, einschliesslich der Getreide-Zellen, unter Anwendung der direkten Gentransformation. In gleicher Weise ist es möglich, die Transformation von Plastiden und Mitochondrien jeder beliebigen anderen Kulturpflanze durchzuführen, wie z.B. Pflanzen der Gattung Solanum, Nicotiana, Brassica, Beta, Pisum, Phaseolus, Glycine, Helianthus, Allium, Triticum, Hordeum, Avena, Setaria, Oryza, Cydonia, Pyrus, Malus, Rubus, Fragaria, Prunus, Arachis, Secale, Panicum Saccharum, Coffea, Camellia, Musa, Ananas, Vitis, Sorghum, Helianthus oder Citrus.

Der Einbau transformierter Gene in Plastide oder Mitochondrien lässt sich mit Hilfe üblicher Methoden nachweisen, z.B. durch genetische Kreuzungsexperimente oder mit molekularbiologischen Nachweisverfahren, insbesondere dem Nachweisverfahren nach Southern für Plastide-und Mitochondrien DNA sowie dem Enzymaktivitäts-Tests.

Das Nachweisverfahren nach Southern kann beispielsweise folgendermassen durchgeführt werden: Die DNA, die aus Plastiden oder Mitochondrien transformierter Zellen oder Protoplasten isoliert wurde, wird nach Behandlung mit Restriktionsenzymen in einem 1 %igen Agarose-Gel einer Elektrophorese unterworfen, anschliessend auf eine Nitrocellulose-Membran überführt [Southern et al., - (1975)] und mit einer in vitro markierten DNA, deren Existenz festgestellt werden soll, hybridisiert. Die DNA kann in vitro mit einer spezifischen Aktivität zwischen $5 \times 10^8$ und $10 \times 10^8$ c.p.m./Mikrogramm mit Hilfe der "nick translation" - [Rigby et al., (1977)] markiert werden. Die Filter werden anschliessend dreimal für eine Stunde mit einer wässrigen Lösung einer 0.03 M Natriumcitrat Lösung und einer 0.3 M Natriumchlorid-Lösung bei einer Temperatur von 65°C gewaschen. Die hybridisierte DNA wird durch eine ein-bis mehrtägige Autoradiographie auf einem Röntgen-Film sichtbar gemacht.

Die Zellen, die mit dem gewünschten Gen transformiert sind, werden mit Hilfe von an sich bekannten Methoden isoliert. Diese Methoden beinhalten Selektion und Screening. Die Selektion nuklearer Gene wird bei Fraley et al., (1983); Herrera-Estrella et al., (1983); und Bevan et al., (1983) beschrieben. Ein Screening kann auf $\beta$-Galaktosidase [Helmer et al., (1984)], auf Nopalin-Synthase oder Octopin-Synthase [Wostemeyer et al., (1984); De-Greve et al., (1982)] oder auf Atrazin-Resistenz erfolgen.

Vor dieser Erfindung wusste man nicht, dass Plastide oder Mitochondrien durch direkten Gentransfer transformiert werden können. Dementsprechend war es vorher nicht möglich brauchbare Gene in Form isolierter DNA funktionsfähig in diese Organellen einzuführen. Gene werden als funktionsfähig in das Genom von Plastiden oder Mitochondrien eingebaut betrachtet, wenn sie eingebaut zur Replikation und Expression fähig sind. Plasmide und Mitochondrien all jener Pflanzen, die aus Protoplasten regenerierbar sind, können ebenfalls erfolgreich mit Hilfe des erfindungsgemässen Verfahrens transformiert werden. Bis zum heutigen Zeitpunkt war es bisher nicht möglich, Plastide und Mitochondrien von Vertretern aus der Familie der Gramineae (Gräser), die ebenso Getreidearten, wie z.B. Mais, Weizen, Reis, Gerste, Hafer, Hirse, Roggen und Sorghum umfassen, genetisch zu manipulieren. Die vorliegende Erfindung erlaubt nunmehr die genetische Transformation von Plastiden und Mitochondrien von Gramineen-Zellen, einschliesslich der genannten Getreide-Zellen, unter Anwendung der direkten Gentransformation. In gleicher Weise ist es möglich die Transformation von Plastiden und Mitochondrien jeder beliebigen anderen Kulturpflanze durchzuführen, wie z.B. Pflanzen der Gattung Solanum, Nicotiana, Brassica, Beta, Pisum, Phaseolus, Glycine, Helianthus, Allium, Triticum, Hordeum, Avena, Setaria, Oryza, Cydonia, Pyrus, Malus, Rubus, Fragaria, Prunus, Arachis, Secale, Panicum, Saccharum, Coffea, Camellia, Musa, Ananas, Vitis, Sorghum, Helianthus oder Citrus.

Der Einbau tranformierter Gene in Plastide oder Mitochondrien lässt sich mit Hilfe üblicher Methoden nachweisen, z.B. durch genetische Kreuzungsexperimente oder mit molekularbiologischen Nachweisverfahren, insbesondere dem Nachweisverfahren nach Southern für Plastid-und Mitochondrien-DNA sowie dem Enzymaktivitäts-Test.

Ein Vorteil bei der Einführung von Genen in Plastide oder Mitochondrien durch direkten Gentransfer liegt in der Möglichkeit der gleichzeitigen Inaktivierung unerwünschter Gene begründet, die bereits im Plastid-oder Mitochondriengenom vorhanden sind. Dies ist deshalb möglich, weil der Einbau fremder Gene ins Plastid-oder Mitochondriengenom bei Anwendung des direkten Gentransfers nicht notwendigerweise über eine homologe Rekombination verläuft. So wird beispielsweise ein Donor-DNA-Molekül, das eine Atrazin-resistente Form von psbA trägt nicht notwendigerweise an der Stelle, wo sich das entsprechende Atrazinsensitive Gen befindet, integriert. Da aber auf der anderen Seite die Plastid-und Mitochondriengenome relativ klein sind, ist es durchaus möglich die transformierten Pflanzenzellen zu selektieren und diejenigen herauszufinden, bei denen die Donor-DNA zufälligerweise in irgendeine gewünschte Funktion des Empfänger-Genoms eingebaut wurde. Der direkte Gentransfer in Plastid-und Mitochondriengenome eröffnet daher auch einen Weg zu Inaktivierung von Genen, der bisher nicht durchführbar war. Mit Hilfe eines entsprechenden Screenings ist es somit beispielsweise möglich, tranformierte Pflanzenzellen zu finden, in denen das Atrazin-sensitive psbA Gen durch den Einbau einer Donor-DNA, die ein Atrazin-Resistenzgen trägt, inaktiviert ist.

Ein weiterer Vorteil des direkten Gentransfers in die Genome von Plastiden und Mitochondrien liegt darin, dass diese Genome, im Gegensatz zu nuklearen Genomen, maternal durch das Cytoplasma vererbt werden und daher gewöhnlich nicht durch Pollen auf ihre sexuellen Nachkommen übertragen werden. Daher wird die Möglichkeit einer Uebertragung von Genen, die für Kulturpflanzen wünschenswerte Merkmale vermitteln, von Kultur-Pflanzen auf Unkräuter stark herabgesetzt.

Pflanzenzellen in Zellkulturen, die mit Hilfe des erfindungsgemässen Verfahrens transformiert werden, können gegebenenfalls für die Produktion von Polypeptiden verwendet werden, die von den inserierten Genen kodiert werden. Solche Synthese-Produkte schliessen beispielsweise das 32 kd Polypeptid ein, das von psbA exprimiert wird, weiterhin die Chloramphenicol-Acetyltransferase, die Glutathion-S-Transferase und die grosse Untereinheit der Rubisco.

Die Pflanzenzellen, die die eingebauten Gene enthalten, können in manchen Fällen auch zu multizellulären Pflanzen regeneriert werden, die das Gen exprimieren und daher die gewünschte neue Eigenschaft besitzen. Jede beliebige Pflanze, die aus Pflanzenzellen, die sich ihrerseits wieder aus Protoplaten ableiten, regenerierbar ist, kann mit Hilfe des erfindungsgemässen Verfahrens hergestellt werden.

Beispiele derartiger, ganzer Pflanzen sind: Solanum spp. (Kartoffel), Petunia spp. (Petunie), Daucus spp. (Karotte), Lycopersicon spp. (Tomate), Brassica spp. (Rübe, Kohl, Blumenkohl etc.), Medicago spp. (Alfalfa), Trifolium spp. (Klee), Zitruns, Atropa, Hyosxyamus, Salpiglossis, Arabidopsis, Digitalis, Cichorium, Gossipium, Glycine, Geranium, Anthirrhinum und Asparagus ein. Die Pflanzen nach an sich bekannten Methoden regeneriert werden; siehe Evans und Bravo, (1983); Dale, (1983); Pflanzen können beispielsweise aus irgendeinem geeigneten Ableger regeneriert werden, wie Zellen, Kalli, Gewebe, Organe, Knospen, Stecklinge, Schösslinge, Wurzelfasern, Pflänzchen, somatischen Embryonen und andere.

Die vorliegende Erfindung betrifft ferner Photoplasten, Pflanzenzellen, Zellaggregate, Pflanzen und insbesondere die Samen von Pflanzen, die mit Hilfe des erfindungsgemässen Verfahren hergestellt wurden, und zwar so lange, wie die gebildeten Samen das eingebaute Gen und damit die daraus resultierende gewünschte Eigenschaft enthalten. Sie betrifft auch alle Nachkommen von Pflanzen, die mit Hilfe des erfindungsgemässen Verfahrens hergestellt wurden, sowohl sexuelle als auch vegetative Nachkommen.

Sexuelle Nachkommen können durch Selbst- oder Fremdbestäubung erhalten werden, wobei Nachkommen auch alle Kreuzungs-und Fusionsprodukte mit dem im vorangehenden Paragraphen definierten, transformierten pflanzlichen Material einschliessen, sofern diese Nachkommen die durch die Transformation eingeführte Eigenschaft aufweisen. Samen und Nachkommen herbizid resistenter oder toleranter Eltern, die eine instabile Herbizid-Resistenz oder Toleranz besitzen, können ihre Herbizid-Resistenz oder Toleranz so lange aufrecht erhalten, solange sie in Gegenwart der Herbizide wachsen. Ein bevorzugtes Herbizid ist das Atrazin. Die vorliegende Erfindung erlaubt somit die Herstellung genetisch manipulierter Pflanzen, die in der Lage sind, eine Behandlung mit Herbiziden, wie z.B. mit Atrazin, in Kozentrationen, die lethal sind für sensitive Pflanzen, zu überleben.

Beispiele:

Einzelne Verfahrensmassnahmen der nun folgenden Beispiele können in allgemeiner Form bei Maniatis et al., (1983) nachgelesen werden. Die Enzyme können von New England Biolabs bezogen werden und sind mit Ausnahme der im Text speziell genannten Abweichungen gemäss den Richtlinien des Herstellers zu verwenden.

Beispiel 1: Konstruktion von pCAT° (vgl. Abb. 1)

1) Das Vektor Plasmid pMON 187, das ein Km^r Gen von Tn 903 trägt, wird mit HindIII und BamHI verdaut.

2) Ein promotor-freies CAT Gen wird als Gel-gereinigtes HindIII/BamHI Fragment des Plasmids pSOV [Gorman et al., (1982)] isoliert.

3) Das Zusammenfügen des Fragments von Schritt 2) mit dem Fragment von Schritt 1) liefert ein Plasmid, das in E.coli HB101 transformiert wird. Die Selektionierung erfolgt auf AP^r. Eine Km^s Kolonie besitzt das Plasmid mit der in Abbildung 1 angegebenen Struktur für pCAT°.

Beispiel 2: Alternative Konstruktion von pCAT°

Beispiel 2 kann mit einem anderen Plasmid als pMON 187 wiederholt werden. Ein geeignetes Plasmid, welches das Km^r Gen von Tn 903 besitzt, kann folgendermassen konstruiert werden:

Ein 1.2 AvaII Fragment, welches das Km^r-Gen von Tn 903 enthält, wird von dem Plasmid pAO2 - [Oca et al., (1982)] isoliert. Die Enden des AvaII Fragmentes werden mit Hilfe der Klenow-Polymerase aufgefüllt und es werden BamHI Linker mit den glatten Enden der Fragmente verknüpft. Die DNA wird anschliessend mit den Restriktionsenzymen TaqI und BamHI behandelt und in das Plasmid pBR327 eingespleisst, welches zuvor mit ClaI und BamHI verdaut wurde. Recombinanten, die das Tn903 Fragment besitzen, übertragen Km^r auf E.coli.

Beispiel 3: Konstruktion von p32CAT (vgl. Abb. 1)

Die folgenden Arbeitsschritte werden durchgeführt um den psbA Promotor mit dem promotorfreien CAT° Gen zu verknüpfen.

4) pCAT° wird mit SmaI und HindIII verdaut

5) Ein Gel-gereinigtes 161 bp SmaI/HindIII Fragment, das den psbA Promotor enthält wird aus dem Plasmid pAH484 isoliert. Das rekombinante Plasmid pAH484 enthält das 3.68 kb EcoRI Fragment einer Chlorplasten DNA aus (Atrazin-) Herbizid-resistenten Amaranthus hybridus, die in pBR322 kloniert wurde. [Hirschberg und McIntosch, (1983)].

6) Das in Schritt 5 isolierte Fragment wird mit dem grösseren Fragment, das aus Schritt 4 hervorgeht, verbunden. Die Verknüpfung führt zu p32CAT, das Cm^r auf transformierte E.coli-Zellen überträgt.

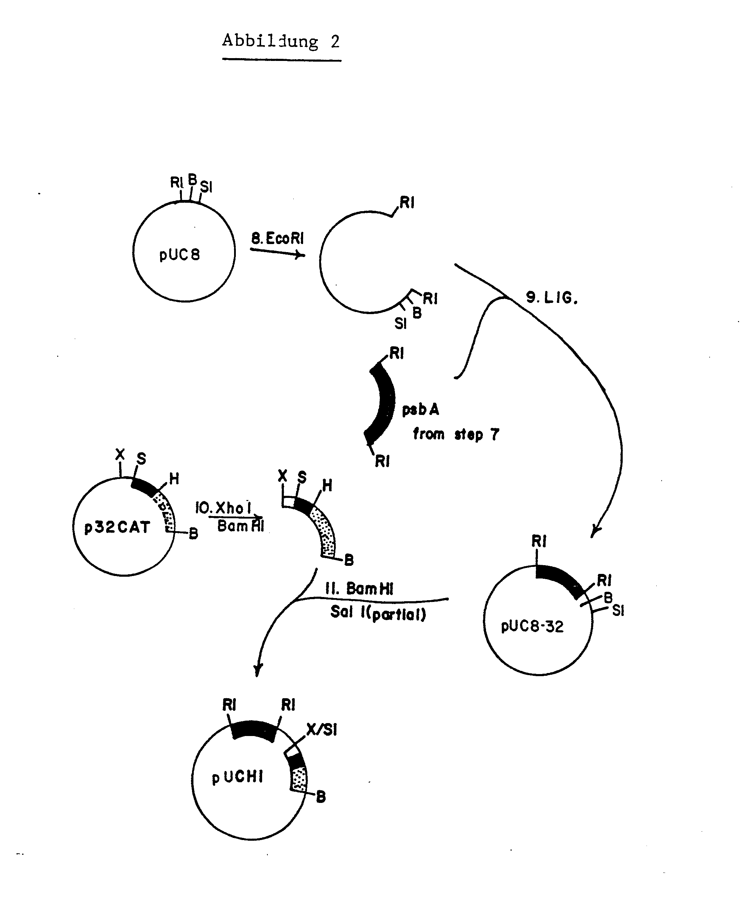

Beispiel 4: Konstruktion von Plasmid pUCH1, ein Vektor mit einem psbA Gen und einer chimären psbA/CAT Gen-Konstruktion (vgl. Abb. 2)

Das Plasmid pUCH1 wird in 5 Schritten in dem pUC8-Vektor konstruiert. (Schritte 7 bis 11):

7) (Abb. 1) Ein 3.6 kb EcoRI Fragment (Gel-gereinigt), welches das komplette psbA Gen enthält wird von dem oben beschriebenen Plasmid pAH484 isoliert.

8) pUC8 (Abb. 2) wird an seiner einzigen EcoRI-Stelle linearisiert.

9) Das linearisierte pUC8 Fragment aus Schritt 8) wird mit dem in Schritt 7) beschriebenen Fragment verknüpft. Die Transformation von E.coli ergibt Ap$^r$-Kolonien, die das gewünschte, eingebaute Fragment besitzen, wobei eine dieser Kolonien mit pUC8-32 bezeichnet wird.

10) Ein Gel-gereinigtes 1.8 kb XhoI/BamHI Fragment wurde von p32Cat isoliert.

11) Das aus Schritt 9) resultierende Plasmid, pUC8-32 wird teilweise mit SalI und vollständig mit BamHI verdaut. Die Verknüpfung mit dem aus Schritt 10) resultierenden Fragment und Selektionierung für Cm$^r$ nacherfolgter Transformation von E.coli, führt zu einer E.coli Kolonie, die pUCH1 enthält.

Beispiel 5: Konstruktion von pBRCAT, das ein chimäres psbA/CAT Gen enthält

Die Konstruction von pBRCAT wird durch Verknüpfung der folgenden drei DNA-Fragmente erreicht:

a) dem EcoRI/HindIII (Promotor) Fragment - (ca. 660 bp) aus dem psbA Gen im Plasmid pAH484 (Schritt 12);

b) dem HindIII/BamHI Fragment von pSVO mit einem promotorfreien CAT Gen (Schritt 2; und

c) dem durch EcoRI/BamHI verdauten pBR322 als Vektor (Schritt 13).

14) Die Verknüpfung führt zu einem Plasmid, pBRCAT, das Cm$^r$ auf E.coli Wirtszellen überträgt, in welches dieses Plasmid durch Transformation eingeschleust wird.

Beispiel 6: Einschleusung von Donor-DNA in pflanzliche Protoplasten

Tabak-Protoplasten mit einer Populationsdichte von $2 \cdot 10^6$ pro ml werden in 1 ml eines K$_3$-Mediums [vgl. Z.Pflanzenphysiologie 78, 453-455 (1976); Mutation Research 81, 165-175 (1981)] das 0.1 mg/Liter 2.4 Dichlorphenoxyessigsäure, 1.0 mg/Liter 1-Naphthylessigsäure und 0.2 mg/Liter 6-Benzylaminopurin enthält suspendiert. Protoplasten werden aus einer Enzymsuspension durch Flotation auf 0.6 M Sucorose bei einem pH von 5.8 und anschliessender Sedimentation 5 Minuten bei 100 g in 0.17 M Calciumchlorid bei pH 5.8 erhalten. Zu dieser Suspension werden nacheinander 0.5 ml 40 %iges ·Polyethylenglykol (PEG) mit einem Molekulargewicht von 6000 in modifiziertem (nach dem Autoklavieren wieder auf einen pH-Wert von 5.8 eingestellt) F-Medium [Nature 296, 72-74 (1982)], sowie 65 Mikroliter einer wässrigen Lösung mit 15 Mikrogramm Donor-DNA (p32CAT, pUCH1, oder pBRCAT) und 50 Mikrogramm Kalbsthymus-DNA hinzugefügt. Dieses Gemisch wird 30 Minuten bei 26°C kultiviert, wobei die Lösung gelegentlich bewegt und anschliessend schrittweise mit F-Medium verdünnt wird. Die Protoplasten werden durch Zentrifugieren (5 Minuten bei 100 g) isoliert und anschliessend in 30 ml frischem K$_3$-Medium resu-spendiert. Die weitere Inkubation erfolgt bei 24°C im Dunkeln in 10 ml Portionen in Petri-Schalen mit einem Querschnitt von 10 cm.

Nach drei Tagen wird das Kulturmedium in jeder Petri-Schale mit 0.3 Volumenteilen eines fri-schen K$_3$-Mediums verdünnt und für weitere 4 Tage bei 24°C und 3000 lux inkubiert. Nach insgesamt 7 Tagen werden die Klone, die sich aus den Protoplasten entwickelt haben, in ein mit 1 %iger Agarose verfestigtes Kulturmedium eingebettet, das 10 mg/Liter Chloramphenicol enthält und bei 24°C in Dunkelheit nach der "bead type culture method" - [Plant Cell Report, 2, 244-247 (1983)] kultiviert. Das Kulturmedium wird alle 5 Tage durch ein frisches Medium der selben Art ersetzt. Nach 3 bis 4 Wochen ununterbrochener Kultivierung in Chloramphenicol-haltigem Kultur-Medium, werden die resistenten Kalli von 2 bis 3 mm Durchmesser auf ein mit Agar verfestigtes LS Kultur-Medium - [Physiol. Plant. 18, 100-127 (1965)], das 0.05 mg/Liter 2.4-Dichlorphenoxyessigsäure, 2mg/Liter 1-Naphthylessigsäure, 0.1 mg/Liter 6-Benzylaminopurin, 0.1 mg/Liter Kinetin und 10 mg/Liter Chloramphenicol enthält, übertragen. Chloramphenicol resistente Nicotiana tabacum Petit Havana SRI Pflanzen erhält man durch Sprossinduktion auf LS-Medium mit 10 ml/Liter Chloramphenicol und 0.2 mg/Liter 6-Benzylaminopurin und anschliessender Wurzelinduktion auf T-Medium [Science 163, 85-87, (1969)].

Die Selektion Chloramphenicol resistenter Kalli und Pflanzen erfolgt im wesentlichen entsprechend den bei De Block et al., (1984) gemachten Angaben.

Beispiel 7: Screening auf Atrazin-resistente Chloroplasten-Transformanten

a) Kalli

Eine Atrazin Toxizitäts-Kurve wird für nicht-transformierte Kalli in einem Konzentrationsbereich von 1 bis 10 Mikromol Atrazin und verschiedenen Lichtintensitäten erstellt. Mutmasslich von Chlorplasten-Transformanten stammender Kalli und Kontroll-Kalli werden auf Nährager-Platten aufgetragen, mit Atrazin-Konzentrationen und Lichtintensitäten, die das Chlorophyll vollständig aus den Kontrollgeweben herausbleichen, behandelt. Die Resistenz gegenüber Atrazin wird visuell, aufgrund der anhaltenden Ergrünung resistenter Gewebe, geprüft.

b) Ganze Pflanzen

Pflanzen, die von Chloroplasten-Transformanten erhalten wurden, werden nach den folgenden Methoden auf Atrazin-Resistenz geprüft:

Chlorophyll-Fluoreszenz-Induktion in Blättern. Wird der Elektronentransport auf der reduzierenden Seite von Photosystem II gehemmt, wie beispielsweise durch Atrazin, dann wird die Chlorophyll absorbierte Strahlungs-Energie als Fluoreszenz reemittiert. Diese Fluoreszenz kann auf der Blattoberfläche gemessen werden. Die Fluoreszenzmessung wird an Blattscheibchen, die horizontal über ein transparentes, Lucite-Fenster gespannt sind, wie bei Nalkin et al., (1981) beschrieben, vorgenommen. [Lucite steht für polymerisiertes Harz aus Methylmethacrylat.] Das Erreger-Licht stammt von einem dc getriebenen Projektor, das, nach Passage eines Filters, in einem Band von aktinischem Licht von 500 bis 600 nm mit Intensitäten um 10 nE x cm$^{-2}$x s$^{-1}$ resultiert. Die Dauer des Erreger-Lichtes beträgt 3 ms. Das Erreger-Licht ist senkrecht zur Blattoberfläche ausgerichtet und die Fluoreszenz von derselben Oberfläche wird mit Hilfe eines flexiblen Lichtführungssystems gesammelt und anschliessend nach Passage eines Cut-off Filters - (660 nm) auf einen rotempfindlichen Photomultiplier übertragen. Die Fluoreszenzdurchgänge werden auf einem Oszilloskop aufgezeichnet und direkt verfilmt.

c) Licht modulierte Flotation

Lässt man ausgeschnittene Blattstückchen auf einem Phosphat-Puffer haltigen Detergenz aufschwimmen, bleiben sie an der Oberfläche, solange die Photosynthese anhält, die für ein hohes O$_2$/CO$_2$ Verhältnis in den Interzellularräumen sorgt.

Lässt man die Blattstückchen dagegen im Dunkeln aufschwimmen oder fügt man dem Medium Photosynthese hemmende Herbizide bei, dann verlieren sie sehr schnell ihren Auftrieb und gehen unter. Eine Assay-Methode für Atrazin-Resistenz ist bei Hensley (1981) beschrieben. Blattstückchen werden in Röhrchen mit Atrazin haltiger Lösung gegeben und unter Vakuum gesetzt. Die Blattstückchen werden schnell von der Lösung durchdrungen und sinken auf den Grund der Lösung. Das Vakuum wird anschliessend aufgehoben, eine Bicarbonat Lösung wird zugegeben und die Röhrchen dem Licht ausgesetzt. Wird die Photosynthese nicht durch Atrazin gehemmt, dann stellt der photosynthetisch erzeugte Sauerstoff innerhalb des Gewebes die Schwimmfähigkeit wieder her und die Blattstückchen schwimmen zur Oberfläche. Atrazin-sensitive Stückchen verbleiben dagegen am Boden.

Beispiel 8: Transformation von Zellen von Brassica rapa (vgl. Just Right)

Brassica rapa Protoplasten werden mit einem geeigenten Osmotikum gewaschen und in einer Populationsdichte von 5•10$^5$ pro ml in einem Kultur-Medium suspendiert, das entsprechend den Anweisungen in [Protoplast 83, Proceedings Experientia Supplementum, Birkhäuser Verlag, Basel, Vol. 45 (1983, 44-45] hergestellt wird. 40 %iges Polyethylenglykol (PEG) mit einem Molekuargwicht von 6000, gelöst in modifiziertem F-Medium (pH 5.8) (vgl. Beispiel 1b), wird mit der Protoplastensuspension bis zu einer Endkonzentration von 13 % PEG vermischt. Zu diesem Gemisch wird anschliessend sofort eine Lösung bestehend aus 50 Mikrogramm des mit Endonuklease Sal I verdauten Plasmids pBRCAT oder p32CAT und 60 Mikrogramm Wasser hinzugefügt. Unter gelegentlichem Rühren wird diese Mixtur 30 Minuten bei 20° bis 25°C inkubiert. Dann werden dreimal 2 ml modiziertes F-Medium (insgesamt 6 ml) und zweimal 2 ml Kultur-Medium (insgesamt 4 ml) in 5 Minuten-Intervallen hinzugegeben. Die Protoplastensuspension wird auf Petri-Schalen mit 10 cm Querschnitt übertragen und mit zusätzlichem Kultur-Medium auf ein Gesamtvolumen von 20 ml ergänzt. Diese Protoplastensuspensionen werden 45 Minuten bei 26°C im Dunkeln inkubiert.

Die Protoplasten werden durch 5-minütige Sedimentation bei 100 g isoliert, in einem zunächst flüssigen, später durch Agarose Gel verfestigten Kultur-Medium aufgenommen und nach der "bead type culture method" [Plant Cell Reports, 2, 244-247 (1983)] kultiviert. Nach vier Tagen, im Entwicklungsstadium der ersten Zellteilung, wird Chloramphenicol in einer Konzentration von 10 mg/Liter zu den Kulturen hinzugegeben. Das flüssige Kultur-Medium, das die Agarose-Segmente umgibt, wird alle vier Tage durch frische, Chloramphenicol haltige Nährlösung ersetzt. Nach vier Wochen werden die Chloramphenicol-resistenten Klone isoliert und anschliessend weiter kultiviert, indem sie wöchentlich mit Chloramphenicol-haltiger Nährlösung versorgt werden (10 mg/Liter).

### Beispiel 9: Transformation von Protoplasten

Man geht aus von Lolium multiflorum-Protoplasten mit einer Konzentration von $2\bullet10^6$ pro ml in einer 0.4 molaren Mannitol-Lösung von pH 5.8. Zu dieser Suspension werden nacheinander 0.5 ml 40 % Polyethylenglycol (PEG) mit einem Molekulargewicht von 6000 in modifiziertem (pH 5.8) F-Medium [Nature 296, 72-74, (1982)] und 65 Mikroliter einer wässrigen Lösung mit 50 Mikrogramm des Plasmids p32CAT oder pBRCAT hinzugefügt. Dieses Gemisch wird 30 Minuten bei 26°C und gelegentlichem Umrühren inkubiert und anschliessend mit F-Medium verdünnt. Dies geschieht gemäss Beschreibung in [Nature 296, (1982)]. Die Protoplasten werden durch Zentrifugation (5 Minuten bei 100 g) isoliert und in 4 ml CC-Kultur-Medium - [Potrykus, Harms und Lörz, Callus Formation from Cell Culture Protoplasts of Corn (Zea Mays L.), Theor. Appl. Genet. 54, 209-214 (1979)] aufgenommen und bei 24°C im Dunkeln inkubiert. Nach 14 Tagen werden die sich entwickelnden Zellkulturen in das gleiche Medium überführt, das aber nunmehr Chloramphenicol (10 mg/Liter) enthält. Resistente Kolonien werden auf ein Agar-Medium -das gleiche Medium wie oben, 10 mg/Liter Chloramphenicol ohne Osmotikum -, übertragen und anschliessend, nachdem sie eine Grösse von mehreren Gramm Frischgewicht pro Kolonie erreicht haben, im Hinblick auf die Gegenwart des bakteriellen Gens und die biologische Aktivität des Gens analysiert.

### Beispiel 10: Transformation von kultivierten Zellen von Nicotiana tabacum durch Uebertragung von p32CAT, pBRCAT oder pUCHI mit Hilfe der Elektroporation

Protoplasten werden erhalten durch Sedimentation von 50 ml einer 10 g-Phasen Suspensionskultur einer Nitrat Reduktase defekten Variante von Nicotiana tabacum, Zell-Stamm nia-115 [Müller, A.J. und R. Grafe, Mol. Gen. Genet. 161, 67-76 - (1978)] und Resuspension in 20 ml einer Enzym-Lösung [2 % Cellulase Onozuka R-10, 1 % Macerozyme RO19 und 0.5 % Driselase (erhältlich von der Chemischen Fabrik Schweizerhalle, Basel), in einer Wasch-Lösung (0.3 M Mannitol, 0.04 M Calciumchlorid und 0.5 % 2-(N-morpholin)-ethansulfonsäure) eingestellt auf pH 5.6 mit KOH] und Inkubation für 3 Stunden auf einer Rundschüttelmaschine bei 24°C. Die Protoplasten werden anschliessend durch Filtration durch ein Sieb mit einer Maschenweite von 100 Mikrometern von unverdautem Gewebe abgetrennt. Das gleiche Volumen einer 0.6 M Sucrose-Lösung wird zugegeben und die erhaltene Suspension 10 Minuten bei 100 g zentrifugiert. Die Protoplasten, die an der Oberfläche aufschwimmen werden gesammelt und dreimal durch Sedimentation in der Wasch-Lösung gewaschen.

Die Transformation wird mit Hilfe der Elektroporation durchgeführt. Die Kammer eines Dialog® "Porators" (erhältlich von Dialog GmbH, Harffstr. 34, 4000 Düsseldorf, Bundesrepublik Deutschland) wird durch Waschen mit 70 %igen Alkohol und anschliessend mit 100 %igem Alkohol sterilisiert und durch einen Strom steriler Luft aus einem Gebläse mit laminarer Luftströmung getrocknet. Die Protoplasten werden in einer Konzentration von $1\bullet10^6$ /ml in einer 0.4 M Mannitol-Lösung suspendiert und mit Magnesiumchlorid auf einen Widerstand von 1.4 kOhm eingestellt. Anschliessend wird pBRCAT, pUCHI oder p32CAT in einer Konzentration von 10 Mikrogramm/ml zugegeben. 0.38 ml Proben dieser Protoplasten-Suspension werden dreimal in Intervallen von 10 Sekunden mit einer Spannung von 1000 oder 2000 Volt beaufschlagt. Die Protoplasten werden anschliessend in einer Konzentration von $1\bullet10^5$/ml in 3 ml eines AA-CH Mediums - [AA Medium of Glimelius, K. et al., Physiol. Plant. 44, 273-277 (1978)], kultiviert, welches sowohl durch Erhöhung der Inositolkonzentration auf 100 mg/Liter und der Sucrose-Konzentration auf 34 g/Liter, als auch durch Zusatz von 0.05 ml/Liter 2-(3-Methyl-2-butenyl)adenin modifiziert wurde und das aufgrund seines Gehalts von 0.6 % Agarose - [Sea Plaque, FMC Corp., Marine Colloids Division, P.O. Box 308, Rockland, Maine 04841, USA] verfestigt ist. Nach einer Woche wird die Agarose-Schicht, die die Protoplasten enthält, in 30 ml eines flüssigen AA-CH Mediums überführt, das 10 mg/Liter Chloramphenicol enthält. Nach drei Wochen, in deren Verlauf jeweils die Hälfte des Mediums wöchentlich durch frisches Medium der gleichen Zusammensetzung ersetzt wird, können die transformierten Zellkolonien visuell wahrgenommen werden. Vier Wochen nach der Ueberführung in das Chloramphenicol-haltige Medium werden diese Kolonien auf ein AA-Medium [Glimelius, K. et al., Physiol. Plant. 44, 273-277 (1978)]; mit 0.8% Agar übertragen, das 10 mg/Liter Chloramphenicol enthält, für die weitere Kultivierung und Untersuchung.

Analoge Untersuchungen mit Protoplasten von Brassica rapa und Lolium multiflorum führen ebenfalls zu erfolgreichen Transformationen.

Beispiel 11: Transformation von Chloroplasten von Nicotiana tabacum Zellen durch Transfer der Donor-DNA mit Hilfe der Elektroporation

Die Präparation des Elektroporators und der Protoplasten erfolgt gemäss Beispiel 10, bzw. Beispiel 6.

Für die Transformation werden Protoplaten von Nicotiana tabacum in einer Konzentration von $1.6 \cdot 10^6$/ml in einer Mannitol-Lösung resuspendiert - [0.4 M, gepuffert mit 0.5 % w/v 2-(N-Morpholin)-ethansulfonsäure; pH 5.6]. Die Widerstand der Protoplasten-Suspension wird in der Porator-Kammer (0.38 ml) gemessen und mit einer Magnesiumchlorid-Lösung auf einen Wert zwischen 1 und 1.2 kOhm eingestellt. 0.5 ml Proben werden entnommen und kommen in mit einem Deckel verschliessbares Plastik-Röhrchen (5 ml Volumen), in die zuvor 40 Mikroliter Waser mit 8 Mikrogramm Donor-DNA und 20 Mikrogramm Kalbthymus-DNA gegeben wurden, sowie 0.25 ml einer Polyethylenglykol-Lösung (24 % w/v in 0.4 M Mannitol).

9 Minuten nach der Zugabe der DNA werden 0.38 ml Proben in die Elektroporator-Kammer gegeben. 10 Minuten nach Zugabe der DNA wird die Protoplastensuspension in der Kammer mit drei Impulsen (1000-2000 Volt) in einem Abstand von 10 Sekunden beaufschlagt. Die behandelten Proben werden in Petrischalen mit 6 cm Durchmesser gegeben und 10 Minuten bei 20°C gehalten. Anschliessend werden 3 ml $K_3$-Medium mit 0.7 % w/v "Sea Plaque Agarose" zu jeder Petrischale zugegeben und der Inhalt der Schale sorgfältig gemischt. Nach der Verfestigung des Inhalts jeder Schale werden die Kulturen einen Tag bei 24°C Dunkeln und 6 Tage im Hellen gehalten. Die Protoplastenhaltige Agarose wird dann in vier Teile zerschnitten und in ein flüssiges Medium eingebracht. Die Protoplasten werden anschliessend nach der "bead type culturing method" kultiviert. Kalli-Gewebe, das durch Selektion des transformierten Materials mit Chloramphenicol erhalten wird und Pflanzen, die daraus regeneriert werden enthalten das CAT-Enzym (Chloramphenicol-Acetyl-Transferase) als Produkt des CAT-Gens.

Die Elektroporation führt zu einer 5 bis 10-fachen Erhöhung der Transformations-Frequenz verglichen mit der Methode ohne Elektroporation. Analoge Untersuchungen mit Brassica rapa c.v. Just Right und Lolium multiflorum ergaben ebenfalls einen Anstieg der Transformations-Frequenz in der gleichen Grössenordnung.

Beispiel 12: Transformation von Zellen von Nicotiana tabacum durch die Uebertragung des CAT-Gens mit Hilfe der Hitze-Schock-Behandlung

Protoplasten, die aus Blättern oder von Nicotiana tabacum Zellkulturen isoliert wurden, werden, wie in den Beispielen 6 und 10 beschrieben, gewonnen und analog den vorhergehenden Beispielen in ein osmotisch stabilisiertes Medium überführt.

Die Protoplasten-Suspensionen werden 5 Minuten bei 45°C gehalten, und dann 10 Sekunden mit Eis auf 0°C abgekühlt; anschliessend wird das Plasmid pBRCAT, pUCHI oder pBRCAT, wie in den Beispielen 6 und 10 beschrieben, hinzugefügt.

Die Hitze-Schock-Behandlung erhöht die Transformations-Frequenz um den Faktor 10 oder mehr, verglichen mit einer Transformation, die ohne diese Behandlung durchgeführt wird.

Beispiel 13: Transformation von Pflanzenzellen verschiedener Herkunft durch Uebertragung des CAT-Gens, indem man in einem ersten Schritt Protoplasten und Gene zusammenbringt und anschliessend eine kombinierte Behandlung durchführt

Pflanzen-Protoplasten: Nicotiana tabacum c.v. Petit Havana SRI (A); Brassica rapa c.v. Just Right (B) und Lolium multiflorum (C) werden isoliert und gemäss Beispiel 11 auf ein osmotisch stabilisiertes Medium übertragen. Die Protoplasten-Suspensionen werden mit dem Plasmid pUCH1, p32CAT oder pBRCAT entsprechend den Beispielen 6 bis 9 vermischt aber ohne gleichzeitige Behandlung mit Polyethylenglykol. Die Protoplastensuspensionen werden dann gemäss Beispiel 12 einer Hitzeschockbehandlung ausgesetzt und anschliessend einer Polyethylenglykol-Behandlung entsprechenden Beispielen 6 bis 9 und zuletzt einer Elektroporation gemäss Beispiel 11 unterworfen. Die Transformationsfrequenz bei dieser Vorgehensweise liegt zwischen $10^{-3}$ und $10^{-2}$, kann je nach den gewählten Bedingungen aber auf 1 % bis 2 % gesteigert werden.

Literaturverzeichnis

Barton, et al, Cell. 32, 1033 (1983).
Barton and Chilton, Methods in Enzymology, 101, 527 (1984).
Bevan, et al, Nature, 304, 184 (1983).
Bevan, et al, Nucleic Acid Res., 12, 8711 (1984).
Bolivar, et al, Gene 2 , 75 (1977).
Chilton, et al, Genetics, 83, 609 (1976).
Chilton, "Plant Gene Vectors", in "The Role of Plant Biotechnology in Plant Breeding", Report des 1984 Plant Breeding Research Forums, 21. bis 23 August (1984), Seiten 177 bis 192 (1985).

Coleman, et al, Cell, 37, 429 (1984).

Comai, et al, Plasmid, 10, 21 (1983).

Dale, "Protoplast Culture and Plant Regeneration of Cereals and Other Recalcitrants Crops" in Protoplasts 1983 Lecture Proceedings, Potrykus et al, - (eds), Experientia Supplement, Band 46, Birkhaeuser, Basel, (1983).

De Block, et al, EMBO J., 4, 1367 (1985).

De Block, et al, EMBO J., 3, 1681 (1984).

De Greve, et al, Nature, 300, 752 (1982).

de Framond, et al, Biotechnology, 1, 266 (1983).

Ditta, et al, Proc. Natl. Acad. Sci USA 77, 7347 - (1980).

Evans and Bravo, "Protoplast Isolation and Culture" im Handbook of Plant Cell Culture, Band 1, Evans et al (Eds), McMillian Publishing Co., New York, - (1983).

Fraley, et al, Proc. Natl. Acad. Sci., 80, 4803 - (1983).

Fraley, et al, Biotechnology, 3, 629 (1985).

Fraley, et al, Plant Molecular Biology, 3, 371 - (1984).

Gamborg, Plant Physiol., 45, 372 (1970).

Gamborg, et al, Plant Tissue Culture Methods, 11 - (1975).

Garfinkel et al, Cell, 27, 143 (1981).

Gorman, et al, Molecular and Cellular Biology, 2, 1044 (1982).

Helmer, et al, Biotechnology, 2, 520 (1984).

Hensley, Weed Sci., 29 70 (1981).

Hepburn, et al, J. Mol Appl. Genet., 2, 211, (1983).

Hernalsteens, et al, Nature, 287, 654 (1980).

Herrera-Estrella, et al, Nature, 303, 209 (1983).

Hirschberg and McIntosch, Science, 222, 1346 - (1983).

Hirschberg, et al, Z. Naturforsch., 396, 412 (1984).

Hoekema, et al, Nature, 303, 179 (1983).

Holsters et al, Mol. Genet., 163, 181 (1978).

Horsch, et al, Science, 227, 1229 (1985).

Izart, et al, Cell, 36, 1007 (1984).

Jordan and Ogren, Nature, 291, 513 (1981).

Jorgensen, et al, Mol Gen. Genet, 177, 65 (1979).

Klee, et al, Biotechnology, 3, 637 (1985).

Koblitz, Kulturpflanze, XXII, 93 (1974).

Le Baron and Gressel, Herbicide Resistance in Plants, John Wiley and Sons, (1982).

Maliga, Z., Pflanzenphysiol., 78, 453 (1976).

Malkin, et al, Plant Physiol., 67, 570 (1981).

Maniatis, et al, Molecular Cloning, Cold Springs Harbor Laboratory, (1982).

Matzke and Chilton, J. Mol Appl. Genet., 1, 39 - (1981).

Messing, et al, Gene, 19, 269 (1982).

Meister, et al, Ann Rev. Biochem., 52, 711, (1983).

Melton, Proc. Natl. Acad. Sci., 82, 144 (1985).

Murashige, et al, Physiol. Plant, 15, 473 (1962).

Neumann, et al, EMBO J., 7, 841 (1982).

Norrander, et al, Gene 26, 101 (1983).

Oka, et al, Nature, 276, 845 (1978).

Oka, et al, J. Mol. Biol., 147, 217 (1981).

Paszkowski, et al, EMBO J., 3 , 2717 (1984).

Pestka, et al, Proc. Natl. Acad. Sci., 81 , 7525 - (1984).

Rennenberg, Phytochemistry 21, 2771 (1982).

Rigby et al, J. Mol. Biol., 113, 237 (1977).

Roberts, Plant Molecular Biology Reporter, 3, 107 - (1985).

Rochaix, et al, Plant Mol. Biol., 3, 363 (1984).

Schilperoort et al, Europäische Patentanmeldung Nr. 86,537 (1983).

Shimabukuro, et al, Plant Physiol., 47, 10 (1971).

Simons, et al, Cell, 34, 683 (1983).

Southern, et al, J. Mol. Biol., 98, 503 (1975).

Wang, et al, Cell, 38, 455 (1984).

Wostemeyer, et al, Mol. Gen., 194, 520 (1984).

Yadav, et al, Proc. Natl. Acad. Sci. USA, 79, 6322 (1982).

Zambryski, et al, EMBO J., 2, 2143, (1983).

## Ansprüche

1. Verfahren zur direkten Einschleusung von DNA in die Plastide und Mitochondrien von pflanzlichen Protoplasten, wobei besagte DNA aus einem oder mehrerer Gene und in Plastiden und Mitochondrien aktiven Promotoren besteht, dadurch gekennzeichnet, dass man in Abwesenheit eines Pathogens diese besagte DNA in einem Medium, in dem die DNA in die Protoplasten und die darin befindlichen Plastide und Mitochondrien einzudringen vermag, so lange mit den Protoplasten in Kontakt bringt, dass diese Penetration gewährleistet ist.

2. Verfahren nach Anspruch 1, dadurch gekennzeichnet, dass man es bis zur Regeneration von transformierten Pflanzenzellen aus den transformierten Protoplasten weiterlaufen lässt.

3. Verfahren nach einem der Ansprüche 1 oder 2, dadurch gekennzeichnet, dass man das Verfahren bis zur Regeneration von transformierten Pflanzen weiterlaufen lässt.

4. Verfahren nach Anspruch 1, dadurch gekennzeichnet, dass es sich bei der einzuschleusenden DNA um lineare DNA handelt.

5. Verfahren nach Anspruch 1, dadurch gekennzeichnet, dass es sich bei der einzuschleusenden DNA um zirkuläre DNA handelt.

6. Verfahren nach Anspruch 5, dadurch gekennzeichnet, dass die zirkuläre DNA keine oder eine noch mehrere T-DNA-Grenzregionen enthält.

7. Verfahren nach Anspruch 1, dadurch gekennzeichnet, dass es sich bei der einzuschleusenden DNA um eine DNA handelt, die in den Plastiden oder Mitrochondrien eine Herbizidresistenz überträgt.

8. Verfahren nach Anspruch 7, dadurch gekennzeichnet, dass es sich bei besagtem Herbizid um Atrazin handelt.

9. Verfahren nach Anspruch 7, dadurch gekennzeichnet, dass die einzuschleusende DNA neben der Herbizidresistenz eine zweite für die Landwirtschaft brauchbare Eigenschaft überträgt.

10. Verfahren nach Anspruch 1, dadurch gekennzeichnet, dass die einzuschleusende DNA einen selektierbaren Marker und ein Gen enthält, welches eine landwirtschaftlich bedeutungsvolle Eigenshaft überträgt.

11. Verfahren nach Anspruch 10, dadurch gekennzeichnet, dass das Markergen eine Antibiotika-Resistenz erzeugt.

12. Verfahren nach Anspruch 11, dadurch gekennzeichnet, dass es sich bei der Antibiotika-Resistenz um Chloramphenicol-oder Kanamycin-Resistenz handelt.

13. Verfahren nach Anspruch 10, dadurch gekennzeichnet, dass es sich bei der landwirtschaftlich bedeutungsvollen Eigenschaft um eine Herbizid-Resistenz handelt.

14. Verfahren nach Anspruch 13, dadurch gekennzeichnet, dass es sich bei der Herbizid-Resistenz um Atrazin-Resistenz handelt.

15. Verfahren nach Anspruch 1, dadurch gekennzeichnet, dass es sich bei dem einzuschleusenden Gen um ein chimäres Gen handelt.

16. Verfahren nach Anspruch 1, dadurch gekennzeichnet, dass die einzuschleusende DNA ein Replikationssignal enthält.

17. Verfahren nach Anspruch 1, dadurch gekennzeichnet, dass die einzuschleusende DNA ein Integrationssignal enthält.

18. Verfahren nach Anspruch 1, dadurch gekennzeichnet, dass man das Verfahren mit aus Blättern stammenden Protoplasten durchführt.

19. Verfahren nach Anspruch 1, dadurch gekennzeichnet, dass man ein osmotisch stabilisiertes für Protoplasten geeignetes Kulturmedium einsetzt.

20. Verfahren nach Anspruch 1, dadurch gekennzeichnet, dass man ein Medium verwendet, das pflanzenverträgliche divalente Kationen enthält.

21. Verfahren nach Anspruch 20, dadurch gekennzeichnet, dass es sich bei den Kationen um Magnesium-oder Calzium-Kationen handelt.

22. Verfahren nach Anspruch 1, dadurch gekennzeichnet, dass das Medium einen mehrwertigen Alkohol enthält, der die Zellmembran verändert und die Zellfusion begünstigt.

23. Verfahren nach Anspruch 22, dadurch gekennzeichnet, dass es sich bei dem mehrwertigen Alkohol um Polyethylenglykol, Polypropylenglykol oder Polyvinylglykol handelt.

24. Verfahren nach Anspruch 23, dadurch gekennzeichnet, dass es sich bei dem mehrwertigen Alkohol um Polyethylenglykol (PEG) handelt.

25. Verfahren nach Anspruch 24, dadurch gekennzeichnet, dass das Polyethylenglykol ein Molekulargewicht zwischen 1000 und 10000 aufweist.

26. Verfahren nach Anspruch 1, dadurch gekennzeichnet, dass die DNA und die Protoplasten einer Hitzeschockbehandlung unterworfen werden.

27. Verfahren nach Anspruch 1, dadurch gekennzeichnet, dass die DNA und die Protoplasten einer Elektroporation unterworfen werden.

28. Verfahren nach Anspruch 1, dadurch gekennzeichnet, dass die Einführung der DNA in die Protoplasten durch Kombination zweier Massnahmen ausgewählt aus einer Polyethylenglykolbehandlung, Hitzeschockbehandlung und Elektroporation erfolgt.

29. Verfahren nach Anspruch 28, dadurch gekennzeichnet, dass der Gentransfer derart durchgeführt wird, dass man zur Einschleusung des Fremdgens das besagte Gen und die Protoplasten in eine Lösung gibt und die resultierende Suspension zuerst einer Hitzeschockbehandlung und anschliessend einer Polyethylenglykolbehandlung unterwirft.

30. Verfahren nach Anspruch 1, dadurch gekennzeichnet, dass der Gentransfer derart durchgeführt wird, dass man zur Einschleusung des Fremdgens das besagte Gen und die Protoplasten in eine Lösung gibt und die resultierende Suspension zuerst einer Hitzeschockbehandlung dann einer Polyethylenglykolbehandlung, und zuletzt einer Elektroporation unterwirft.

31. Verfahren nach Anspruch 1, dadurch gekennzeichnet, dass das verwendete Medium einen mehrwertigen Alkohol enthält, der die Protoplastenmembran verändert und die Zellfusion fördert und man die in diesem Medium suspendierte DNA zusammen mit den Protoplasten einer Elektroporation und/oder einer Hitzeschockbehandlung unterwirft.

32. Verfahren nach Anspruch 1, dadurch gekennzeichnet, dass man zusätzlich die extracellulären Nukleasen inaktiviert.

33. Verfahren nach Anspruch 1, dadurch gekennzeichnet, dass man Protoplasten aus Pflanzenzellen der Pflanzen der Familien der Gramineae, Solamaceae oder der Gruciferae einzetzt.

34. Verfahren nach Anspruch 33, dadurch gekennzeichnet, dass man Protoplasten aus Pflanzenzellen der Pflanzen der Familie der Gramineae einsetzt.

35. Verfahren nach Anspruch 34, dadurch gekennzeichnet, dass es sich bei den Gramineae um Getreide handelt.

36. Verfahren nach Anspruch 35, dadurch gekennzeichnet, dass es sich bei dem Getreide um Mais, Weizen, Reis, Gerste, Hafer, Hirse, Roggen oder Sorghum handelt.

37. Die aus dem in den Ansprüchen 1 bis 36 resultierenden transformierten Protoplasten, Pflanzenzellen, Zellaggregate, Pflanzen und Samen sowie deren Nachkommen, die die durch die Transformation erzeugte neue Eigenschaft aufweisen.

38. Alle Kreuzungs-und Fusionsprodukte mit dem in Anspruch 37 definierten transformierten Pflanzenmaterial, die die durch die Transformation erzeugten neuen Eigenschaften aufweisen.

Abbildung 1

Abbildung 2

## Abbildung 3